# EUROPÄISCHE PATENTSCHRIFT

(11) **EP 1 114 117 B1**
(45) Veröffentlichungstag und Bekanntmachung des Hinweises auf die Patenterteilung: **03.11.2004**
(21) Anmeldenummer: 99946029.8
(22) Anmeldetag: 26.08.1999
(51) Int. Cl.: C09K 9/02, G02F 1/15, C07D 401/14

(54) **ELEKTROCHROME VORRICHTUNG MIT GELBFILTER**
ELECTROCHROMIC DEVICE WITH A YELLOW FILTER
DISPOSITIF ELECTROCHROMIQUE DOTE D'UN FILTRE JAUNE

(30) Priorität: 08.09.1998 DE 19840938; 04.05.1999 DE 19920360
(43) Veröffentlichungstag der Anmeldung: 11.07.2001
(73) Patentinhaber: Bayer Chemicals AG, 51368 Leverkusen (DE)
(72) Erfinder: BERNETH, Horst, D-51373 Leverkusen (DE); MICHAELIS, Stephan, D-51519 Odenthal (DE); NEIGL, Ralf, D-51373 Leverkusen (DE); WOMELSDORF, Hermann, Jens, D-51375 Leverkusen (DE)
(86) Internationale Anmeldenummer: PCT/EP1999/006260
(87) Internationale Veröffentlichungsnummer: WO 2000/014172

(56) Entgegenhaltungen:
- WO-A-98/05737
- WO-A-98/35267
- WO-A-99/09112
- US-A- 5 239 405
- US-A- 5 278 693
- US-A- 5 680 245
- DATABASE WPI Section Ch, Week 199008 Derwent Publications Ltd., London, GB; Class E13, AN 1990-054976 XP002123138 & JP 02 008286 A (TOMOEGAWA PAPER MFG CO LTD), 11. Januar 1990 (1990-01-11)
- HAGFELD A ET AL: "NANOSTRUCTURED TIO2 SEMICONDUCTOR ELECTRODES MODIFIED WITH SURFACE ATTACHED VIOLOGENS: APPLICATIONS FOR DISPLAYS AND SMART WINDOWS" PROCEEDINGS OF THE SPIE,US,SPIE, BELLINGHAM, VA, Bd. 2531, Seite 60-69 XP000671702

## Beschreibung

Die vorliegende Erfindung betrifft eine lichtgeschützte elektrochrome Vorrichtung und neue elektrochrome Substanzen.

Elektrochrome Vorrichtungen sind bereits bekannt, beispielsweise aus D. Theis in Ullmann's Encyclopedia of Industrial Chemistry, Vol. A 8, S. 622, Verlag Chemie 1987 und WO-A 94/23333. Man unterscheidet zwei Grundtypen:
Typ 1: vollflächige elektrochrome Vorrichtung.
Typ 2: elektrochrome Anzeigevorrichtungen mit strukturierten Elektroden.

Typ 1 findet beispielsweise bei elektrisch abdunkelbaren Fensterscheiben oder elektrisch abblendbaren Autospiegeln Anwendung. Solche Vorrichtungen sind beispielsweise aus US-A4 902 108 bekannt.

Typ 2 findet bei Segment- und Matrixanzeigen Verwendung. Solche Anzeigevorrichtungen sind beispielsweise in DE-A 196 31 728 vorgeschlagen worden. Derartige Vorrichtungen können transmissiv oder bei Verspiegelung reflektiv betrachtet werden.

In WO-A 94/23333 werden elektrochrome Materialien verschiedener Bauweise gegenübergestellt, die aber nicht als Anzeigevorrichtungen verwendet werden:
- Bauweise a:: Die elektrochromen Substanzen liegen als Film oder Schicht fest auf den Elektroden ( Ullmann, s.o.).
- Bauweise b:: Die elektrochromen Substanzen werden beim Redoxprozeß auf den Elektroden als Schicht abgeschieden ( Ullmann, s.o.).
- Bauweise c:: Die elektrochromen Substanzen bleiben permanent in Lösung.

Für Bauweise a) ist als elektrochromes Material das Paar Wolframoxid/Palladiumhydrid das bekannteste.

Für Bauweise b) sind Viologene als elektrochrome Substanzen beschrieben worden. Diese Vorrichtungen sind nicht selbstlöschend, das erzeugte Bild bleibt also nach dem Abschalten des Stromes bestehen und kann nur durch Umpolen der Spannung wieder gelöscht werden. Solche Vorrichtungen sind nicht besonders beständig und erlauben keine hohe Zahl an Schaltzyklen.

Zudem sind insbesondere solche mit Wolframoxid/Palladiumhydrid aufgebauten Zellen wegen der Lichtstreuung an diesen elektrochromen Schichten nicht im durchfallenden Licht zu betreiben, sondern lediglich reflektiv.

Aus Elektrokhimiya 13, 32-37 (1977), 13, 404-408, 14, 319-322 (1978), US-A 4 902 108 und US-A 5 140 455 ist ein elektrochromes System dieser letztgenannten Bauweise c) bekannt. In einer elektrochromen Zelle, die aus leitfähig beschichteten Glasplatten aufgebaut ist, ist eine Lösung eines Paares elektrochromer Substanzen in einem inerten Lösungsmittel enthalten.

Als Paar von elektrochromen Substanzen wird je eine elektrochemisch reversibel reduzierbare und eine reversibel oxidierbare Substanz verwendet. Beide sind im Grundzustand farblos oder nur schwach gefärbt. Unter Einfluß einer elektrischen Spannung wird die eine Substanz reduziert, die andere oxidiert, wobei beide farbig werden. Nach Abschalten der Spannung bildet sich bei beiden Substanzen der Grundzustand wieder zurück, wobei Entfärbung bzw. Farbaufhellung auftritt.

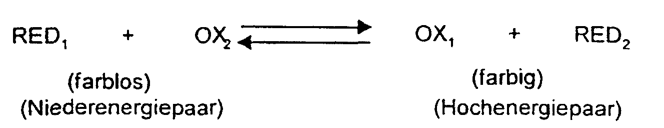

Aus US-A 4 902 108 ist bekannt, daß solche Paare von Redoxsubstanzen geeignet sind, bei denen die reduzierbare Substanz wenigstens zwei chemisch reversible Reduktionswellen im Cyclischen Voltammogramm und die oxidierbare Substanz entsprechend wenigstens zwei chemisch reversible Oxidationswellen besitzt.

Gemäß WO-A 94/23333 haben derartige Lösungssysteme der Bauweise c) jedoch gravierende Nachteile.

Die Diffusion der elektrochromen Substanzen in der Lösung bedingt unscharfe Farbgrenzen und verursacht einen hohen Stromverbrauch zur Aufrechterhaltung des gefärbten Zustandes, da die gefärbten Substanzen durch Rekombination und Reaktion an der jeweils gegenüberliegenden Elektrode permanent abgebaut werden.

Nichtsdestoweniger sind für solche elektrochromen Zellen der Bauweise c) verschiedene Anwendungen beschrieben worden. So können sie beispielsweise als Automobilrückspiegel ausgebildet sein, der bei Nachtfahrt durch Anlegen einer Spannung abgedunkelt werden kann und somit das Blenden durch Scheinwerfer nachfolgender Fahrzeuge verhindert (US-A 3 280 701, US-A 4 902 108, EP-A 0 435 689). Weiterhin können solche Zellen auch in Fensterscheiben oder Auto-Sonnendächem eingesetzt werden, wo sie nach Anlegen einer Spannung das Sonnenlicht abdunkeln. Ebenfalls beschrieben ist die Anwendung solcher Vorrichtungen als elektrochrome Anzeigevorrichtungen, beispielsweise in Segment- oder Matrix-Displays mit strukturierten Elektroden ( DE-A 196 31 728).

Die elektrochromen Zellen bestehen normalerweise aus einem Paar Glasplatten, von denen im Falle des Autospiegels eine verspiegelt ist. Eine Seite dieser Scheiben ist mit einer lichtdurchlässigen, elektrisch leitfähigen Schicht, beispielsweise Indium-Zinn-Oxid (ITO), flächig beschichtet, wobei im Falle der Anzeigevorrichtungen diese leitfähige Beschichtung in elektrisch voneinander getrennte Segmente aufgeteilt ist, die einzeln kontaktiert sind. Aus diesen Scheiben wird nun eine Zelle aufgebaut, indem sie mit ihrer einander zugewandten elektrisch leitfähig beschichteten Seite über einen Dichtungsring zu einer Zelle verbunden werden. In diese Zelle wird nun über eine Öffnung eine elektrochrome Flüssigkeit eingefüllt und die Zelle dicht verschlossen. Über die ITO-Schichten werden die beiden Scheiben mit einer Spannungsquelle verbunden.

Die vorstehend beschriebenen elektrochromen Vorrichtungen zeigen in der Regel eine Empfindlichkeit gegenüber Licht, insbesondere UV-Licht. Es sind deshalb beispielsweise in US-A 5 280 380 elektrochrome Vorrichtungen beschrieben, die UV-Absorber enthalten. Es sind auch elektrochrome Autospiegel beschrieben worden, die solche Absorber in einer Splitterschutz-Beschichtung enthalten (US-A 5 073 012).

Der aus dem Stand der Technik bekannte UV-Schutz bewirkt eine Verbesserung der Lichtbeständigkeit der elektrochromen Vorrichtungen im ungeschalteten, stromlosen Zustand. Für die Anwendung in Automobilrückspiegeln ist dies auch ausreichend, da sie tagsüber bei starker Lichteinwirkung stets ungeschaltet sind und nur nachts bei geringer Lichteinwirkung geschaltet, d. h. abgedunkelt werden.

Für andere Anwendungen elektrochromer Vorrichtungen, beispielsweise Fenster oder Anzeigevorrichtungen, genügt dieser Schutz nicht, da sie gerade bei starker Lichteinwirkung geschaltet werden.

Die Aufgabe der vorliegenden Erfindung bestand darin, die Lichtbeständigkeit von elektrochromen Vorrichtungen im geschalteten Zustand zu verbessern.

Überraschenderweise wurde nun gefunden, daß ein Schutz der elektrochromen Vorrichtung im Wellenlängenbereich von 390 bis 430 nm mittels eines Gelbfilters deren Lichtbeständigkeit im eingeschalteten Zustand erheblich verbessert.

Gegenstand der Erfindung ist demnach eine lichtgeschützte elektrochrome Vorrichtung, enthaltend ein Paar Glas- oder Kunststoffplatten oder Kunststoffolien, von denen mindestens eine Platte oder Folie, vorzugsweise beide Platten oder Folien auf jeweils einer Seite mit einer elektrisch leitfähigen Beschichtung versehen sind, von denen wenigstens eine Platte oder Folie und ihre leitfähige Beschichtung transparent sind, von denen die andere verspiegelt sein kann und von denen wenigstens bei einer der beiden Platten oder Folien die elektrisch leitfähige Schicht in getrennte, einzeln kontaktierte Flächensegmente aufgeteilt sein kann, wobei die Platten oder Folien über einen Dichtungsring auf den Seiten ihrer leitfähigen Beschichtung zusammengefügt sind, und das Volumen, gebildet aus den beiden Platten oder Folien und dem Dichtungsring, mit einem elektrochromen Medium gefüllt ist, dadurch gekennzeichnet, daß die elektrochrome Vorrichtung einen Gelbfilter enthält, bei dem die Wellenlänge, bei der die Extinktion in der langweiligen Flanke die Hälfte des längstwelligen maximalen Extinktionswertes erreicht, zwischen 390 und 430 nm liegt.

Das Absorptionsmaximum liegt vorzugsweise zwischen 390 und 410 nm, ganz besonders bevorzugt zwischen 390 und 405 nm. Ganz besonders bevorzugt sind solche Substanzen und Materialien, bei denen die Halbwertsbreite der Absorptionsbande, d. h. die Breite der Bande bei der Hälfte ihrer Maximalextinktion, kleiner als 100 nm, insbesondere kleiner als 80 nm, ganz besonders kleiner als 60 nm ist. Vorzugsweise ist der langwellige Abfall der Absorptionsbande steiler als der kurzwellige.

Ebenfalls bevorzugt sind auch Materalien, die zusätzlich Wellenlängen unterhalb von 350 nm filtern. Sie haben dann im Bereich 380 bis 420 nm, eine Absorptionskante, unterhalb der starke Absorption und oberhalb der schwache oder vorzugsweise keine Absorption auftritt (Kantenfilter). Solche Materialien können Mischungen aus einzelnen Materialien sein, deren einzelne Absorptionen sich zu dieser breitbandigen Absorption zusammensetzen. Es können aber auch Materialen sein, die diese Eigenschaft allein besitzen. Beispiele sind hier spezielle Gläser oder oxidische oder keramische Beschichtungen sowie insbesondere Nanoteilchen.

Diese Gelbfilter sind in dem elektrochromen Medium enthalten und/oder auf und/oder in wenigstens einer der beiden transparenten Platten oder Folien fixiert. Beispielsweise können die Platten oder Folien mit transparenten Beschichtungen überzogen sein, die diese Gelbfilter enthalten. Solche transparenten Beschichtungen sind beispielsweise Kunststoffe, z. B. Polyurethane, Polyacrylate, Polymethacrylate, Polyamid, Polycarbonat, Polyester, oder anorganische Beschichtungen, beispielsweise auf Silikatbasis, soggenannte Ormocere.
Die Gelbfilter müssen also in dem elektrochromen Medium oder in der Beschichtung oder in der Platte oder Folie löslich oder dispergierbar sein, so daß keine Lichtstreuung auftritt. Sie können auch in der Beschichtung oder in der Platte oder Folie chemisch gebunden sein.

Als Gelbfilter kommen insbesondere kurzwellig absorbierende Gelbfarbstoffe in Frage.

Beispiele sind: 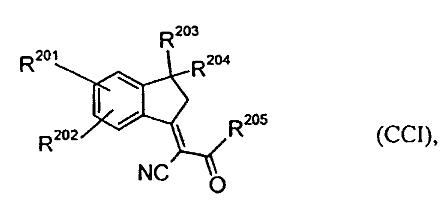 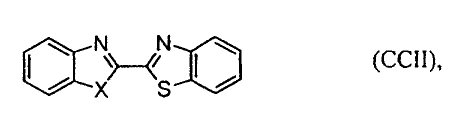 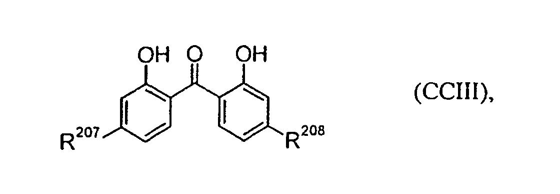 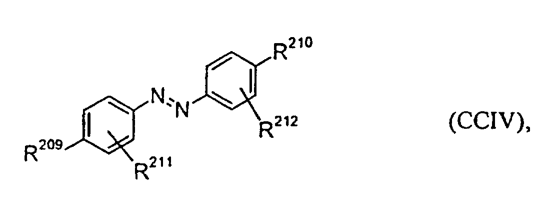 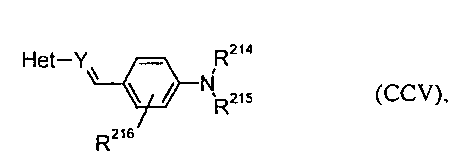 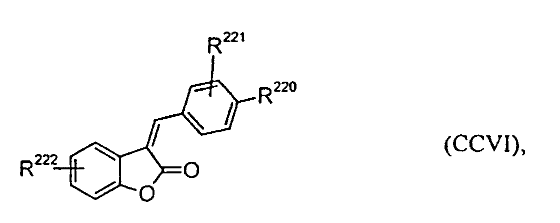 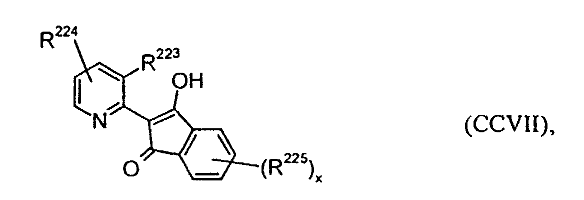 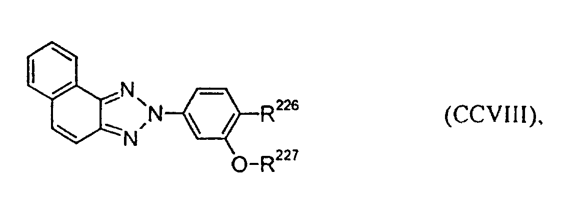 worin
- R²⁰¹ und R²⁰²: unabhängig voneinander für C₁- bis C₈-Alkoxy stehen,
- R²⁰³ und R²⁰⁴: unabhängig voneinander für Wasserstoff oder C₁- bis C₄-Alkyl stehen,
- R²⁰⁵: für C₁- bis C₁₈-Alkyl oder C₁- bis C₁₈-Alkoxy steht,
- X: für S oder N-R²⁰⁶ steht,
- R²⁰⁶ und R²²⁷: unabhängig voneinander für C₁- bis C₁₂-Alkyl stehen,
- R²⁰⁷ und R²⁰⁸: unabhängig voneinander für Hydroxy, C₁- bis C₈-Alkoxy oder C₆- bis C₁₀-Aryloxy stehen,
- R²⁰⁹ und R²¹⁰: unabhängig voneinander für C₁- bis C₈-Alkoxy, C₁- bis C₈-Alkylthio, NR²¹⁷R²¹⁸, C₆- bis C₁₀-Aryloxy, Cyano, CO-OR²¹⁷, CO-NR²¹⁷R²¹⁸, NR²¹⁸-CO-R²¹⁹, NR²¹⁸-SO₂-R²¹⁹ stehen und
- R²⁰⁹: zusätzlich für Wasserstoff oder C₁- bis C₄-Alkyl steht,
- R²¹¹ und R²¹²: unabhängig voneinander für Wasserstoff, Halogen, C₁- bis C₄-Alkyl, C₁- bis C₄-Alkoxy oder NR²¹⁸-CO-R²¹⁹ stehen,
- Het: für Benzthiazol-2-yl, Benzoxazol-2-yl, Benzimidazol-2-yl, Thiazol-2-yl, 1,3,4-Thiadiazol-2-yl, 2- oder 4-Pyridyl, 2- oder 4-Chinolyl oder 3,3-Dimethylindolen-2-yl steht, die durch Methyl, Methoxy, Chlor, Cyano, Nitro, Methoxycarbonyl, Methylthio, Dimethylamino, Diethylamino oder Dipropylamino substituiert sein können.
- Y: für N oder C-R²¹³ steht,
- R²¹³: für Wasserstoff, C₁- bis C₄-Alkyl, Cyano, CO-R²¹⁹, CO-O-R²¹⁷ oder CO- NR²¹⁷R²¹⁸ steht,
- R²¹⁴ und R²¹⁵: unabhängig voneinander für Wasserstoff, C₁- bis C₈-Alkyl, CO-R²¹⁹ oder C₆- bis C₁₀-Aryl stehen oder
- NR²¹⁴R²¹⁵: für Pyrrolidino, Piperidino oder Morpholino steht,
- R²¹⁶: für Wasserstoff, Halogen, C₁- bis C₄-Alkyl, C₁- bis C₄-Alkoxy oder NH-CO- R²¹⁹ steht,
- R²¹⁷ und R²¹⁸: unabhängig voneinander für Wasserstoff, C₁- bis C₈-Alkyl oder C₆- bis C₁₀-Aryl stehen,
- R²¹⁹: für C₁- bis C₈-Alkyl oder C₆- bis C₁₀-Aryl steht,
- R²²⁰ bis R²²²: unabhängig voneinander für Wasserstoff, C₁- bis C₈-Alkyl oder C₁- bis C₈-Alkoxy stehen,
- R²²³: für Wasserstoff oder Hydroxy steht,
- R²²⁴: für Wasserstoff, Halogen oder C₁- bis C₄-Alkyl steht,
- R²²⁵: für Wasserstoff oder Halogen steht,
- x: für eine ganze Zahl von 1 bis 4 steht und
- R²²⁶: für CHO, CN, CO-C₁- bis C₈-Alkyl oder CO-C₆- bis C₁₀-Aryl steht,
wobei die Alkyl-, Alkoxy- und Arylreste gegebenenfalls weitere Reste wie Alkyl, Halogen, Nitro, Cyano, CO-NH₂, Alkoxy oder Phenyl tragen können und die Alkyl- und Alkoxyreste geradkettig oder verzweigt sein können.

Als Gelbfilter kommen insbesondere auch Fluoreszenzfarbstoffe in Frage.

Beispiele sind: 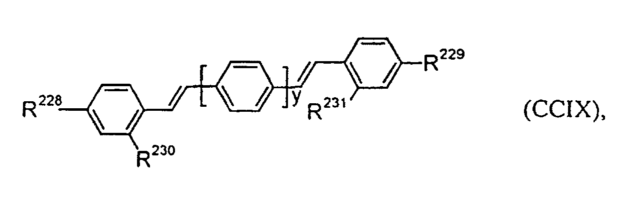 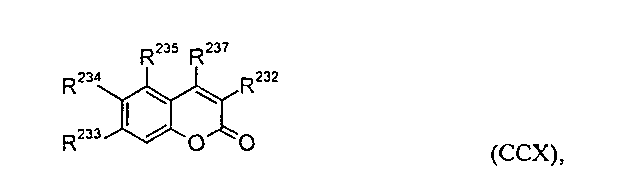 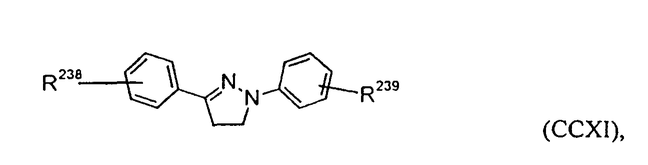 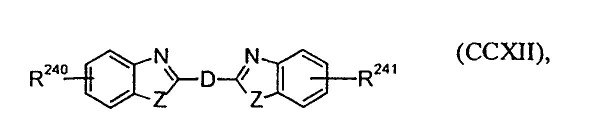 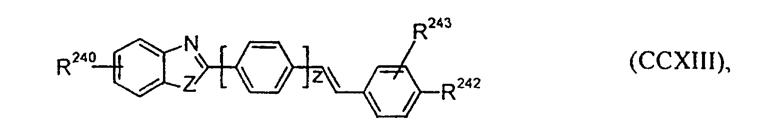 worin
- R²²⁸ bis R²³¹: unabhängig voneinander für Wasserstoff, Halogen, Cyano oder C₁- bis C₄-Alkoxy stehen,
- y: für 1 oder 2 steht,
- R²³²: für Wasserstoff, Cyano, CO-O-C₁-bis C₄-Alkyl, C₆- bis C₁₀-Aryl, Thiophen-2-yl, Pyrid-2- oder 4-yl, Pyrazol-1-yl oder 1,2,4-Triazol-1- oder -4-yl steht,
- R²³³: für Wasserstoff, C₁- bis C₈-Alkoxy, 1,2,3-Triazol-2-yl oder Di-C₁- bis C₄-alkylamino steht,
- R²³⁴ und R²³⁵: für Wasserstoff stehen oder gemeinsam für eine -CH=CH-CH=CH- Brücke stehen,
- R²³⁷: für Wasserstoff, C₁- bis C₄-Alkyl oder Cyano steht,
- R²³⁸ und R²³⁹: unabhängig voneinander für Wasserstoff, Halogen, CO-C₁- bis C₄-Alkyl oder SO₂-C₁- bis C₄-Alkyl stehen,
- R²⁴⁰, R²⁴¹ und R²⁴³: unabhängig voneinander für Wasserstoff oder C₁- bis C₈-Alkyl stehen,
- Z: für O oder N-R²⁴⁴ steht,
- D: für -CH=CH-, 1,4-Phenylen, Naphthalin-1,4-diyl, Thiophen-2,5-diyl oder Furan-2,5-diyl steht,
- R²⁴²: für Wasserstoff, Cyano oder CO-O-C₁- bis C₈-Alkyl steht,
- z: für 0 oder 1 steht und
- R²⁴⁴: für C₁- bis C₈-Alkyl steht,
wobei die Alkyl-, Alkoxy-, Aryl- und heterocyclischen Reste gegebenenfalls weitere Reste wie Alkyl, Halogen, Nitro, Cyano, CO-NH₂, Alkoxy oder Phenyl tragen können, die Alkyl- und Alkoxyreste geradkettig oder verzweigt sein können und die heterocyclischen Reste benzanneliert sein können.

Die Reste R²⁰¹, R²⁰⁵ R²⁰⁶, R²⁰⁷, R²⁰⁹, R²¹¹, R²¹⁴, R²²⁰, R²²⁷, R²³³ und R²⁴⁴ können auch die Bedeutung -P-Q haben,
worin
- P: ein Bindeglied bedeutet und
- Q: eine Gruppierung bedeutet, die in ein Polymer eingebaut werden kann.

Beispielsweise bedeutet P eine -(CH₂)ₚ Gruppierung, wobei p eine ganze Zahl von 1 bis 12 bedeutet, und Q bedeutet beispielsweise OH (für Polyester, Polyurethane, Polyharnstoffe), NH₂ (für Polyharnstoffe oder Polyamide), -O-(CO)-CH=CH₂ oder O-(CO)-C(CH₃)=CH₂ für Poly(meth)acrylate und Copolymere mit anderen olefinischen Monomeren wie Styrol, Acrylnitril, Butadien usw.

Die Absorptionsmaxima der genannten Verbindungen der Formeln (CCI) bis (CCXIII) liegen in Abhängigkeit von den Substituenten beispielsweise in folgenden Bereichen:
(CCI): 350 bis 380 nm, insbesondere 355 bis 380 nm,
(CCII): 350 bis 370 nm, insbesondere 355 bis 370 nm,
(CCIII): 350 bis 360 nm, insbesondere 355 bis 360 nm,
(CCIV): 350 bis 400 nm, insbesondere 355 bis 400 nm,
(CCV): 370 bis 420 nm,
(CCVI): 350 bis 400 nm, insbesondere 355 bis 400 nm,
(CCVII): 380 bis 420 nm,
(CCVIII): 355 bis 390 nm,
(CCIX): 355 bis 390 nm,
(CCX): 360 bis 420 nm,
(CCXI): 360 bis 390 nm,
(CCXII): 355 bis 390 nm,
(CCXIII): 355 bis 390 nm.

Übliche UV-Absorber, wie sie beispielsweise in US-A 5 280 380 und US-A 5 073 012 auf Basis von substituierten Benzophenonen oder Zimtsäureestern beschrieben sind, haben Absorptionsmaxima zwischen 300 bis 350 nm.

Bevorzugt sind Gelbfarbstoffe der Formeln (CCI) bis (CCVIII),
worin
- R²⁰¹ und R²⁰²: unabhängig voneinander für Methoxy, Ethoxy, Propoxy oder Butoxy stehen,
- R²⁰³ und R²⁰⁴: unabhängig voneinander für Wasserstoff, Methyl oder Ethyl stehen,
- R²⁰⁵: für Methyl, Ethyl, Propyl, Butyl, Pentyl, Hexyl, Octyl, Decyl, Hydroxyethyl, Methoxyethyl, Ethoxypropyl, -CH₂CH₂O-(CO)-CH=CH₂, Benzyl, Phenylpropyl, Methoxy, Ethoxy, Propoxy, Butoxy, Pentoxy, Hexoxy, Octoxy, Dccoxy, Hydroxyethoxy, Methoxyethoxy, Ethoxypropoxy, -OCH₂CH₂-O- (CO)-CH=CH₂, Benzyloxy oder Phenylpropoxy steht,
- X: für S oder N-R²⁰⁶ steht,
- R²⁰⁶ und R²²⁷: unabhängig voneinander für Methyl, Ethyl, Propyl, Butyl, Pentyl, Hexyl, Heptyl oder Octyl steht,
- R²⁰⁷ und R²⁰⁸: unabhängig voneinander für Hydroxy, Methoxy, Ethoxy, Propoxy, Butoxy, Benzyloxy oder Phenoxy stehen,
- R²⁰⁹ und R²¹⁰: unabhängig voneinander für Methoxy, Ethoxy, Propoxy, Butoxy, Hexoxy, Methoxyethoxy, Methylthio, Ethylthio, Amino, NHR²¹⁷, Phenoxy, Cyano, CO-OR²¹⁷, CO-NR²¹⁷R²¹⁸, NR²¹⁸-CO-R²¹⁹, NR²¹⁸-SO₂-R²¹⁹ stehen und
- R²⁰⁹: zusätzlich für Wasserstoff, Methyl, Hydroxyethoxy, -OCH₂CH₂-O-(CO)-CH=CH₂, -O-(CH₂)₄-O-(CO)-C(CH₃)=CH₂, -NH-(CO)-C₆H₄-O-CH₂CH₂-O-(CO)-CH=CH₂ steht,
- R²¹¹ und R²¹²: unabhängig voneinander für Wasserstoff, Fluor, Chlor, Brom, Methyl, Ethyl, Methoxy oder Ethoxy stehen,
- Het: für Benzthiazol-2-yl, Benzoxazol-2-yl oder 2- oder 4-Pyridyl steht,
- Y: für C-R²¹³ steht,
- R²¹³: für Wasserstoff, Cyano, CO-R²¹⁹, CO-O-R²¹⁷ oder CO-NR²¹⁷R²¹⁸ steht,
- R²¹⁴ und R²¹⁵: unabhängig voneinander für Methyl, Ethyl, Propyl, Butyl, Hexyl, Chlorethyl, Methoxyethyl, Hydroxyethyl, Cyanethyl, Benzyl, Phenethyl, Phenylpropyl, Phenyl, Tolyl, Methoxyphenyl, Chlorphenyl oder CO-R²¹⁹ stehen und
- R²¹⁴: zusätzlich für Wasserstoff oder CH₂CH₂-O-(CO)-C(CH₃)=CH₂ steht oder
- NR²¹⁴R²¹⁵: für Pyrrolidino, Piperidino oder Morpholino steht,
- R²¹⁶: für Wasserstoff, Fluor, Chlor, Brom, Methyl, Ethyl, Methoxy, Ethoxy oder NH-CO-R²¹⁹ steht,
- R²¹⁷ und R²¹⁸: unabhängig voneinander für Methyl, Ethyl, Propyl, Butyl, Hexyl, Benzyl, Phenethyl, Phenylpropyl, Phenyl, Tolyl, Methoxyphenyl oder Chlorphenyl stehen und
- R²¹⁸: zusätzlich für Wasserstoff steht,
- R²¹⁹: für Methyl, Ethyl, Propyl, Butyl oder Phenyl steht,
- R²²⁰: für Methoxy, Ethoxy, Propoxy oder Butoxy steht,
- R²²¹: für Wasserstoff oder Methoxy steht,
- R²²² und R²²⁴: für Wasserstoff stehen,
- R²²³: für Wasserstoff steht,
- R²²⁵: für Wasserstoff oder Chlor steht,
- x: für 4 steht und
- R²²⁶: für CHO steht und
- R²²⁷: für Butyl, Pentyl, Hexyl, Heptyl oder Octyl steht,
wobei die Alkyl- und Alkoxyreste geradkettig oder verzweigt sein können, z. B. n-Butyl, 2-Butyl, tert.-Butyl.

Besonders bevorzugt sind Gelbfarbstoffe der Formeln (CCI) bis (CCVIII),
worin
- R²⁰¹ und R²⁰²: für Methoxy stehen,
- R²⁰³ und R²⁰⁴: für Methyl stehen,
- R²⁰⁵: für Propyl, Butyl, tert.-Butyl, Propoxy oder Butoxy steht,
- X: für N-(2-Ethyl-1-hexyl) steht,
- R²⁰⁷ und R²⁰⁸: für Hydroxy stehen,
- R²⁰⁹ und R²¹⁰: gleich sind und für Methoxy, Ethoxy, Amino, NH-Methyl, NH-Ethyl, Cyano, CO-O-Methyl, CO-O-n-Butyl, CO-NH-n-Butyl, CO-NH-Phenyl, NH-CO-n-Butyl, NH-CO-tert.-Butyl oder NH-CO-Phenyl stehen,
- R²¹¹ und R²¹²: unabhängig voneinander für Wasserstoff oder Methyl stehen,
- Het: für Benzthiazol-2-yl oder 4-Pyridyl steht,
- Y: für C-R²¹³ steht,
- R²¹³: für Wasserstoff, Cyano, CO-NH₂, Acetyl oder CO-O-Methyl steht,
- R²¹⁴ und R²¹⁵: unabhängig voneinander für Methyl, Ethyl, Butyl, Cyanethyl, Benzyl, Phenyl oder Acetyl stehen,
- R²¹⁶: für Wasserstoff, Methyl oder Methoxy steht,
- R²²⁰ und R²²¹: unabhängig voneinander für Wasserstoff, Methyl, Ethyl, Propyl, Butyl, Methoxy, Ethoxy, Propoxy oder Butoxy stehen,
- R²²² und R²²⁴: für Wasserstoff stehen,
- R²²³: für Wasserstoff oder Hydroxy steht,
- R²²⁵: für Wasserstoff oder Halogen steht,
- x: für 4 steht und
- R²²⁶: für CHO oder CN steht.

Ganz besonders bevorzugt sind Gelbfarbstoffe der Formeln (CCI), (CCV), (CCVI) und (CCVIII).

Ebenfalls bevorzugt sind Fluoreszenzfarbstoffe der Formeln (CCIX) bis (CCXIII),
worin
- R²²⁸ bis R²³¹: unabhängig voneinander für Chlor oder Cyano stehen,
wobei zwei dieser Reste auch für Wasserstoff stehen können,
- y: für 1 steht,
- R²³²: für C₆- bis C₁₀- Aryl, Pyrazol-1-yl, 4-Chlor-pyrazol-2-yl oder 1,2,4-Triazol-1- oder -4-yl steht,
- R²³³: für Wasserstoff, Methoxy, Ethoxy, 4-Phenyl-5-methyl-1,2,3-triazol-2-yl, 4-Ethyl-5-methyl-1,2,3-triaxol-2-yl, Dimethylamino oder Dielhylamino steht,
- R²³⁴ und R²³⁵: für Wasserstoff stehen oder gemeinsam für eine -CH=CH-CH=CH- Brücke stehen,
- R²³⁷ und R²⁴³: für Wasserstoff stehen,
- R²³⁸ und R²³⁹: unabhängig voneinander für Chlor, Acetyl, Propionyl oder Methylsulfonyl stehen,
- R²⁴⁰ und R²⁴¹: unabhängig voneinander für Wasserstoff, Methyl, Ethyl, Propyl oder Butyl stehen,
- Z: für O oder N-R²⁴⁴ steht,
- D: für -CH=CH-, 1,4-Phenylen, Thiophen-2,5-diyl oder Furan-2,5-diyl steht,
- R²⁴²: für Wasserstoff, Cyano oder CO-O-Methyl, -Ethyl, -Propyl oder -Butyl steht,
- z: für 0 oder 1 steht und
- R²⁴⁴: für Methyl, Ethyl, Propyl oder Butyl steht,
wobei die Alkyl- und Alkoxyreste geradkettig oder verzweigt sein können, z. B. n-Butyl, 2-Butyl, tert.-Butyl.

Besonders bevorzugt sind Fluoreszenzfarbstoffe der Formeln (CCIX) bis (CCXIII),
worin
- R²³⁰ und R²³¹: für Cyano stehen,
- y: für 1 steht,
- R²³²: für Phenyl oder 4-Chlor-pyrazol-1 -yl steht,
- R²³³: für Methoxy, 4-Phenyl-5-methyl-1,2,3-triazol-2-yl, 4-Ethyl-5-methyl-1,2,3-triazol-2-yl, Dimethylamino oder Diethylamino steht,
- R²³⁴ und R²³⁵: für Wasserstoff stehen,
- R²²⁸, R²²⁹, R²³⁷ und R²⁴³: für Wasserstoff stehen,
- R²³⁸ und R²³⁹: für Chlor stehen,
- R²⁴⁰ und R²⁴¹: gleich sind und für Wasserstoff, Methyl oder tert.-Butyl stehen,
- Z: für O steht,
- D: für -CH=CH- oder Thiophen-2,5-diyl steht,
- R²⁴²: für CO-O-Methyl steht und
- z: für 0 steht.

Ganz besonders bevorzugt sind die Fluoreszenzfarbstoffe der Formeln (CCIX), (CCX) und (CCXII).

Die Stabilität dieser organischen Gelbfilter gegen Tageslichtexposition kann dadurch gesteigert werden, daß sie mit UV-Absorbern kombiniert werden, die Strahlungen unterhalb 350nm absorbieren.

Insbesondere anorganische Nanoteilchen kommen für diese Aufgabe in Frage.

Als Gelbfilter kommen ebenfalls insbesondere Nanoteilchen in Frage.

Geeignete Nanoteilchen sind solche auf Basis SiC, AlSi, , Fe₂O₃, Fe₃O₄, TiO₂, ZnO, GaP, CeO₂, ZnS, SnO₂, Si_{y}Ge_{1-y}, WₓMo₁₋ₓO₃, NiO, Bi₂O₃, In₂O₃, HfO₂, BaTiO₃, CaTiO₃, Ge, AIP, GaN, worin 0,7≤ y <1 und 0≤ x ≤1 sind.

Im Sinne der Erfindung besonders geeignete Nanoteilchen sind die aus der oben genannten Literatur und den Patentanmeldungen bekannten Materialien auf Basis TiO₂, ZnO, CeO₂, SiC, AlSi, Fe₂O₃, Fe₃O₄, WₓM₁₋ₓO₃, BaTiO₃, CaTiO₃ oder Mischungen davon.

Besonders bevorzugt sind Nanoteilchen mit einem mittleren Durchmesser von kleiner als 500 nm, bevorzugt von kleiner als 100 nm, besonders bevorzugt von kleiner als 50 nm, ganz besonders bevorzugt von kleiner als 20 nm.

Bevorzugt sind Nanoteilchen aus allen bisher beschriebenen Materialien, die kugelförmig oder nahezu kugelförmig sind. Unter nahezu kugelförmig sind beispielsweise Ellipsoide zu verstehen mit einem Achsenverhältnis von bis zu 1:4. vorzugsweise von bis zu 1:2.

Ebenfalls bevorzugt sind Nanoteilchen aus allen bisher beschriebenen Materialien, die eine Kern-Hüllenstruktur aufweisen. Die Hülle kann organisch modifiziert sein.

Die Hülle besteht beispielsweise aus einem Oxid des Materials des Nanoteilchens. Sie kann aber auch aus einem anderen Material bestehen, das im Sichtbaren transparent ist und dessen Brechungsindex dem des Nanoteilchens ähnlich ist. Die Dicke einer Oxidschicht kann beispielsweise zwischen 1 und 300 nm betragen.

In einer bevorzugten Ausführungsform der Erfindung weisen die festen Verbindungen, in denen Silicium im stöchiometrischen Überschuß vorliegt, eine Kern-Hüllen-Struktur auf. Bevorzugt ist dabei, daß dieser aus einem Kern aus Titannitrid und einer Hülle aus Silicium besteht, wobei der Silicium-Volumenanteil mindestens 30 % je Partikel ist.

Die Absorptionskanten von Nanoteilchen liegen beispielsweise im folgenden Bereich:
CeO₂: 380 nm
Si: 380 nm
SiC: 415 nm.

Als Gelbfilter kommen ebenfalls Mischungen aus den Verbindungen der Formeln (CCI) bis (CCXIII) mit Nanoteilchen in Frage.

Um einen durch den Gelbfilter eventuell hervorgerufenen Gelbstich der elektrochromen Vorrichtung zu kompensieren, kann die erfindungsgemäße lichtgeschützte elektrochrome Vorrichtung zusätzlich violette bis blaue Komponenten enthalten. Diese können in dem elektrochromen System und/oder in einer der Platten oder Folien und/oder auf einer der Platten oder Folien enthalten sein. Dabei kann es sich beispielsweise um lösliche, violette oder blaue Farbstoffe handeln.

Die erfindungsgemäßen Fluoreszenzfarbstoffe der Formeln (CCIX) bis (CCXIII) können ihrerseits den Gelbstich der elektrochromen Vorrichtung verringern.

In einer bevorzugten Ausführungsform enthält der erfindungsgemäße Gelbfilter, insbesondere die Nanoteilchen, zusätzlich Partikel, beispielsweise aus Oxiden und/oder Nitriden von Metallen, die im roten Spektralbereich von 600 nm < λ < 700 nm stärker absorbieren als im blau-grünen Spektralbereich von 400 nm < λ < 550 nm. Als solche Zusätze sind Partikel aus Titannitrid mit einem mittleren Durchmesser von 1 nm bis 400 nm, bevorzugt 10 nm bis 120 nm oder Agglomerate aus diesen Titannitrid-Primärpartikeln bevorzugt. Deren Herstellung kann z.B. gemäß US-A 5 472 477 erfolgen.

In einer bevorzugten Ausführungsform enthält der Gelbfilter, insbesondere die Nanoteilchen, neben Silicium-Partikeln auch TiN-Partikel mit einem mittleren Durchmesser von 10 bis 120 nm. Diese Partikel wirken sehr effektiv im UVA-Bereich und gewährleisten gleichzeitig eine Farbneutralität bei hoher Transparenz. Ebenfalls bevorzugt sind Zusätze in Form von Partikeln aus Aluminium-Natrium-Silikaten (Ultramarine Pigmente), beispielsweise erhältlich unter der Bezeichnung Nubix® Pigmente (Firma Nubiola S.A.). Weiterhin können sie als Zusätze Eisen(III)hexacyanoferrat(II) enthalten.

Erfindungsgemäß besonders bevorzugt sind elektrochrome Vorrichtungen, in denen wenigstens eine der beiden leitfähigen Schichten mit einer elektrochromen Schicht überzogen ist, sowie elektrochrome Vorrichtungen, in denen das elektrochrome Medium eine elektrochrome Lösung oder ein Gel darstellt, dadurch gekennzeichnet, daß die elektrochrome Vorrichtung durch einen Gelbfilter vor Licht geschützt ist.

Bevorzugt sind solche erfindungsgemäßen lichtgeschützten elektrochromen Vorrichtungen, in denen
a) die reduzierbare Substanz mindestens eine, vorzugsweise wenigstens zwei chemisch reversible Reduktionswellen im cyclischen Voltammogramm und die oxidierbare Substanz entsprechend mindestens eine, vorzugsweise wenigstens zwei chemisch reversible Oxidationswellen besitzt, oder
b) die reduzierbare Substanz und die oxidierbare Substanz über eine Brücke B kovalent aneinander gebunden sind, oder
c) als reduzierbare und/oder oxidierbare Substanz solche ausgewählt sind, bei denen der reversible Übergang zwischen der oxidierbaren Form und der reduzierbaren Form oder umgekehrt mit dem Bruch bzw. dem Aufbau einer σ -Bindung verbunden ist, oder
d) die reduzierbare Substanz und/oder die oxidierbare Substanz Metallsalze oder Metallkomplexe sind von solchen Metallen, die in mindestens zwei Oxidationsstufen existieren, oder
e) die reduzierbare und/oder oxidierbare Substanz Oligo- und Polymere sind, die mindestens eines der genannten Redoxsysteme, aber auch Paare solcher Redoxsysteme, wie sie unter a) bis d) definiert sind, enthalten, oder
f) als reduzierbare und/oder oxidierbare Substanz Mischungen der in a) bis e) beschriebenen Substanzen eingesetzt werden, vorausgesetzt diese Mischungen enthalten mindestens ein reduzierbares und mindestens ein oxidierbares Redoxsystem.

Durch Auswahl der elektrochromen Verbindungen RED₁ und OX₂ und/oder Mischungen davon lassen sich beliebige monochrome Farbtöne einstellen. Für eine polychrome Farbdarstellung können zwei oder mehrere solcher elektrochromer Vorrichtungen flächig aufeinander gelegt werden, wobei jede dieser Vorrichtungen einen anderen Farbton erzeugen kann. Vorzugsweise wird ein solcher Stapel so aufgebaut, daß die sich berührenden Vorrichtungen eine lichtdurchlässige Platte gemeinsam haben, die dann auch auf beiden Seiten leitfähig beschichtet ist und je nach Ausführung in Segmente unterteilt ist. Beispielsweise besteht dann ein Stapel aus drei elektrochromen Vorrichtungen aus mindestens vier Platten. Durch Einschalten von Segmenten in verschiedenen dieser gestapelten Vorrichtungen lassen sich mehrfarbige Anzeigen realisieren. Werden hintereinander liegende Segmente verschiedener solcher Vorrichtungen eingeschaltet, erhält man Mischfarben. So lassen sich im Rahmen einer Trichromie beliebige Farben darstellen. a!so beispielsweise bunte Bilder.

Im Sinne der Erfindung geeignete OX₂ und RED₁ sind solche Substanzen, die bei ihrer Reduktion bzw. Oxidation an der Kathode bzw. Anode in dem genannten Lösungsmittel Produkte RED₂ und OX₁ liefern, die keine chemische Folgereaktion eingehen, sondern komplett wieder zu OX₂ und RED₁ oxidiert bzw. reduziert werden können.

Vorzugsweise besitzen die elektrochromen Verbindungen RED₁ oder OX₂ in ihrem entsprechenden geschalteten, farbigen Zustand OX₁ bzw. RED₂ neben der eigentlichen, für die Funktion der elektrochromen Vorrichtung wichtigen starken Absorption im sichtbaren Teil des Lichtspektrums eine weitere starke Absorption im Bereich 350 bis 450 nm, also im Übergangsbereich vom ultravioletten zum violetten und blauen Licht.

Geeignete reduzierbare Substanzen OX₂ sind beispielsweise 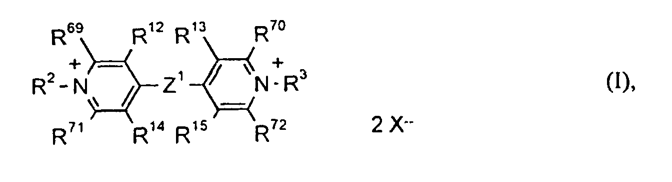 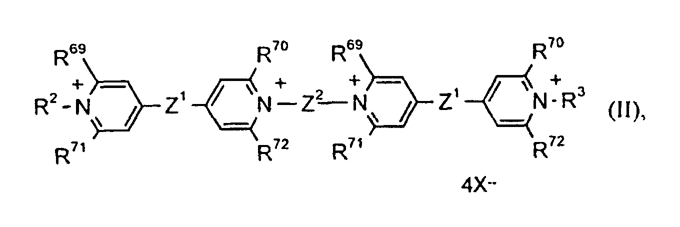 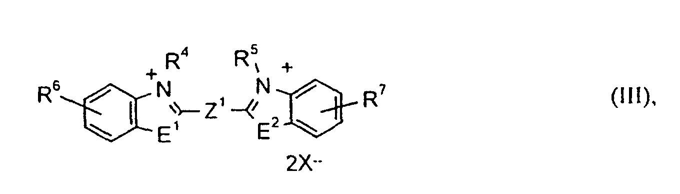 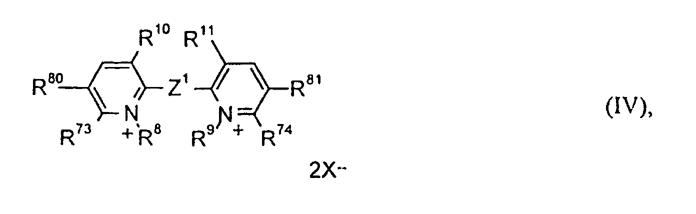 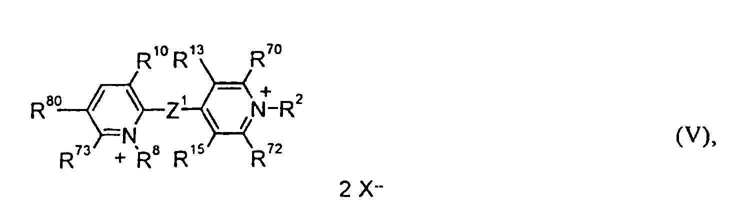 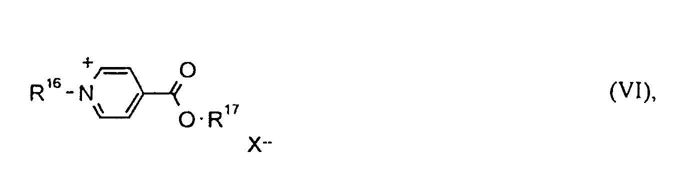 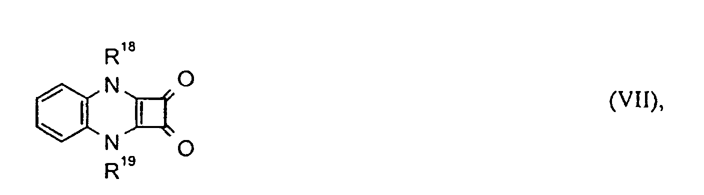 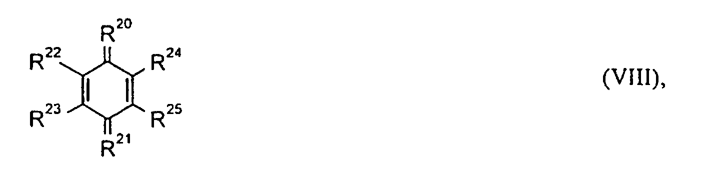 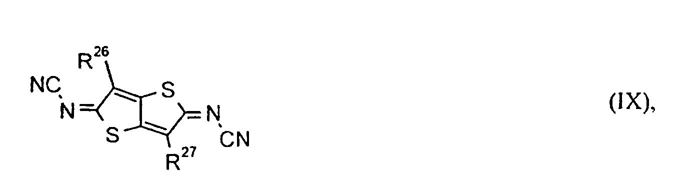 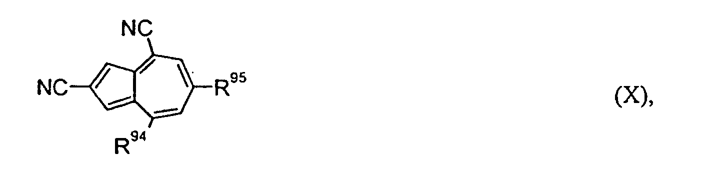 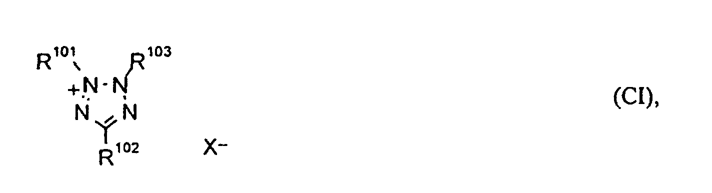 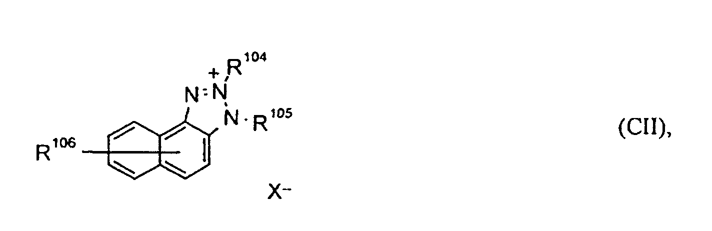 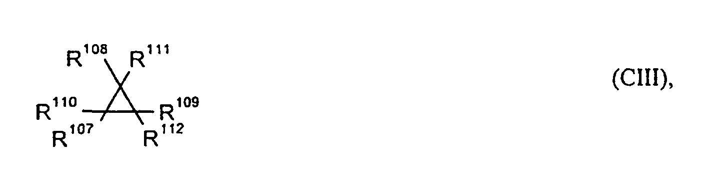 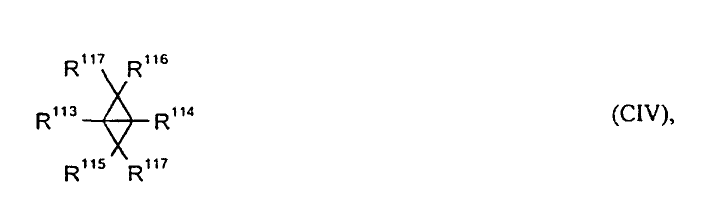 worin
- R² bis R⁵, R⁸, R⁹, R¹⁶ bis R¹⁹: unabhängig voneinander C₁- bis C₁₈-Alkyl, C₂- bis C₁₂-Alkenyl, C₄- bis C₇-Cycloalkyl, C₇- bis C₁₅-Aralkyl oder C₆- bis C₁₀-Aryl bedeuten oder
- R⁴; R⁵ bzw. R⁸; R⁹: gemeinsam eine -(CH₂)₂- oder -(CH₂)₃-Brücke bilden können.
- R⁶, R⁷ und R²² bis R²⁵: unabhängig voneinander Wasserstoff, C₁- bis C₄-Alkyl, C₁- bis C₄-Alkoxy, Halogen, Cyan, Nitro oder C₁- bis C₄-Alkoxycarbonyl bedeuten oder
- R²²; R²³ und/oder R²⁴; R²⁵: eine -CH=CH-CH=CH-Brücke bilden können,
- R¹⁰; R¹¹, R¹⁰; R¹³, R¹²; R¹³ und R¹⁴; R¹⁵: unabhängig voneinander Wasserstoff oder paarweise eine -(CH₂)₂-, -(CH₂)₃- oder -CH=CH-Brücke bedeuten,
- R²⁰ und R²¹: unabhängig voneinander O, N-CN, C(CN)₂ oder N-C₆- bis C₁₀-Aryl bedeuten,
- R²⁶ und R²⁷: Wasserstoff, C₁- bis C₄-Alkyl, C,- bis C₄-Alkoxy, Halogen, Cyan, Nitro, C₁- bis C₄-Alkoxycarbonyl oder C₆- bis C₁₀-Aryl bedeuten,
- R⁶⁹ bis R⁷⁴, R⁸⁰ und R⁸¹: unabhängig voneinander Wasserstoff oder C₁- bis C₆-Alkyl bedeuten oder
- R⁶⁹; R¹², R⁷⁰; R¹³, R⁷³; R⁸⁰ und/oder R⁷⁴; R⁸¹: gemeinsam eine -CH=CH-CH-CH- Brücke bilden,
- E¹ und E²: unabhängig voneinander O, S, NR' oder C(CH₃)₂ bedeuten oder
- E¹ und E²: gemeinsam eine -N-(CH₂)₂-N-Brücke bilden,
- R¹: C₁- bis C₁₈-Alkyl, C₂- bis C₁₂-Alkenyl, C₄- bis C₇-Cycloalkyl, C₇- bis C₁₅-Aralkyl, C₆- bis C₁₀-Aryl bedeutet,
- Z¹: eine direkte Bindung, -CH=CH-, -C(CH)=CH-, -C(CN)=CH-, -CCl=CCl-, -C(OH)=CH-, -CCl=CH-, -C≡C-, -CH=N-N=CH-, -C(CH₃)=N-N=C(CH₃)- oder -CCl=N-N=CCl- bedeutet,
- Z²: -(CH₂)ᵣ- oder -CH₂-C₆H₄-CH₂- bedeutet,
- r: eine ganze Zahl von 1 bis 10 bedeutet,
- R⁹⁴ und R⁹⁵: unabhängig voneinander Wasserstoff oder Cyano bedeuten,
- R¹⁰¹ bis R¹⁰⁵: unabhängig voneinander C₆- bis C₁₀-Aryl oder einen ggf. benzanellierten aromatischen oder quasiaromatischen fünf- oder sechsgliedrigen heterocyclischen Ring bedeuten,
- R¹⁰⁷, R¹⁰⁹, R¹¹³ und R¹¹⁴: unabhängig voneinander einen Rest der Formeln (CV) bis (CVII) 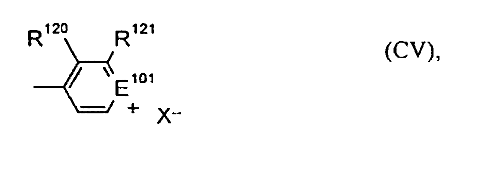 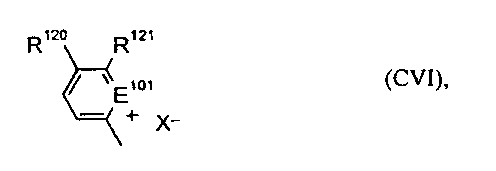 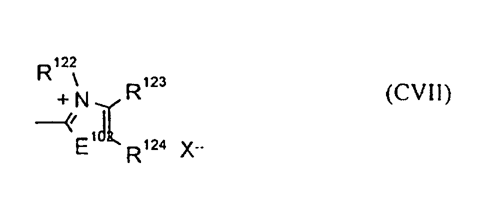 bedeuten,
- R¹⁰⁸, R¹¹⁵ und R¹¹⁶: unabhängig voneinander C₆- bis C₁₀-Aryl oder einen Rest der Formel (CV) bedeuten,
- R¹¹⁰ bis R¹¹², R¹¹⁷ und R¹¹⁸: unabhängig voneinander Wasserstoff, C₁- bis C₄-Alkyl, Halogen oder Cyano bedeuten,
- E¹⁰¹ und E¹⁰²: unabhängig voneinander O, S oder N-R¹¹⁹ bedeuten,
- R¹¹⁹ und R¹²²: unabhängig voneinander C₁- bis C₁₈-Alkyl, C₂- bis C₈-Alkenyl, C₄- bis C₇-Cycloalkyl, C₇- bis C₁₅-Aralkyl oder C₆- bis C₁₀-Aryl bedeuten,
- R¹⁰⁶, R¹²⁰, R¹²¹, R¹²³ und R¹²⁴: unabhängig voneinander Wasserstoff, C₁- bis C₄-Alkyl, C₁- bis C₄-Alkoxy, Halogen, Cyano, Nitro oder C₁- bis C₄-Alkoxycarbonyl bedeuten oder
- R¹²⁰, R¹²¹ bzw. R¹²³, R¹²⁴: gemeinsam eine -CH=CH-CH=CH-Brücke bilden und
- X⁻: ein unter den Bedingungen redox-inertes Anion bedeutet.

Geeignete oxidierbare Substanzen RED₁ sind beispielsweise 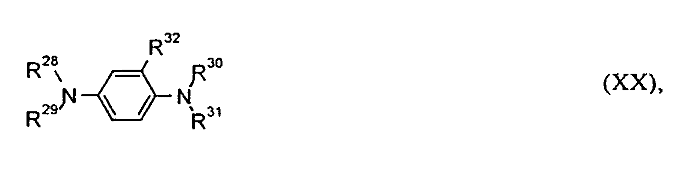 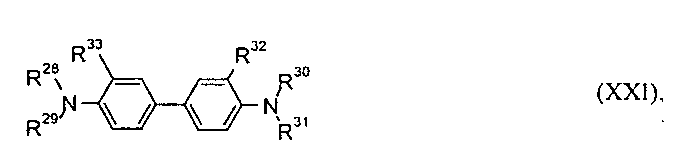 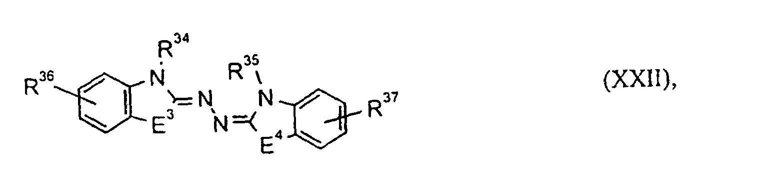 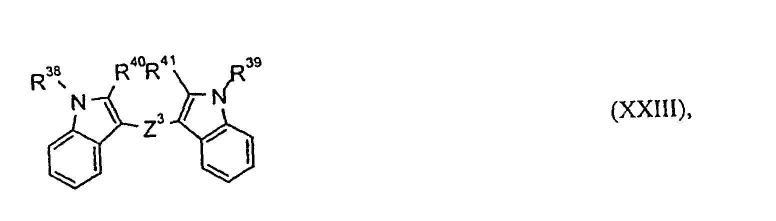 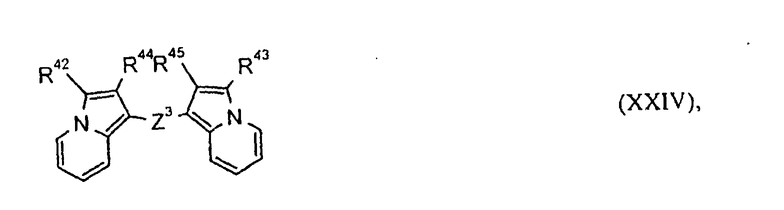 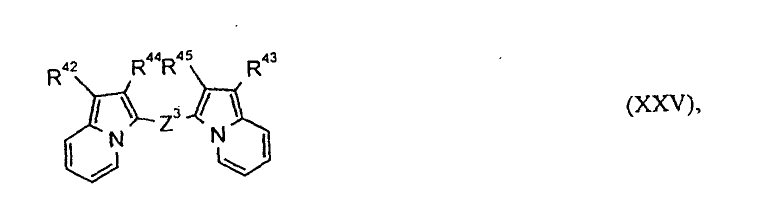 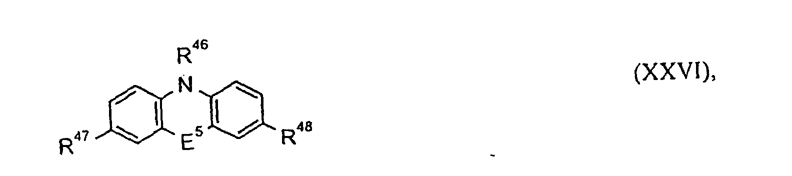 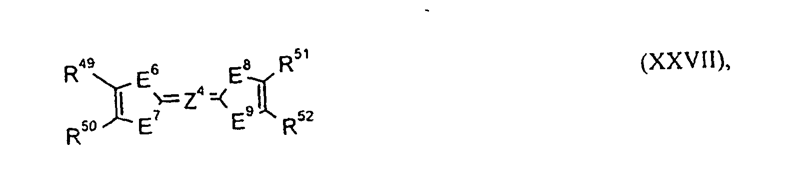 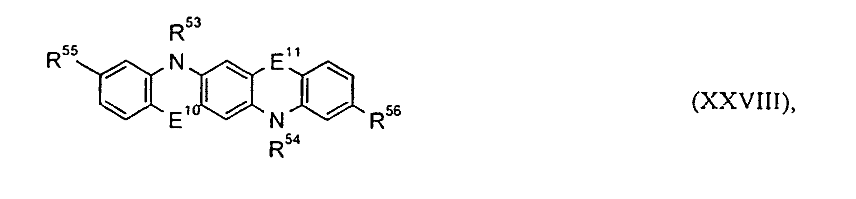 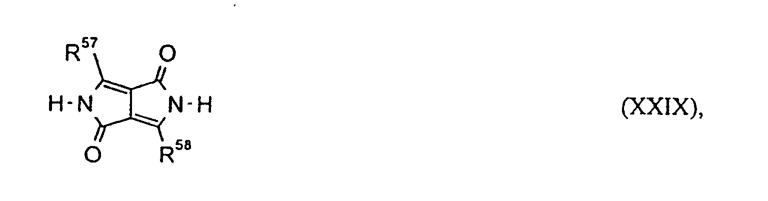 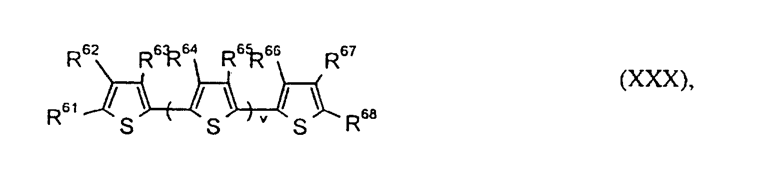 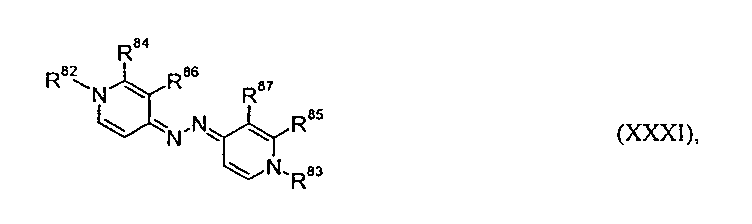 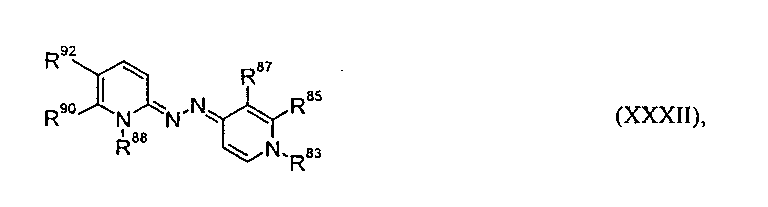 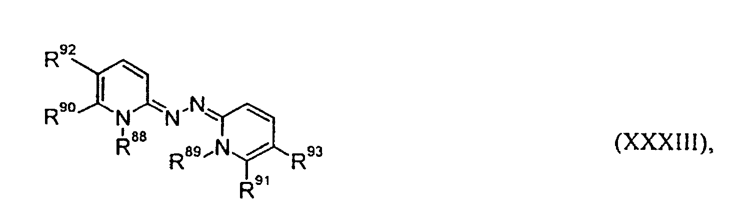 worin
- R²⁸ bis R³¹, R³⁴, R³⁵, R³⁸, R³⁹, R⁴⁶, R⁵³ und R⁵⁴: unabhängig voneinander C₁- bis C₁₈-Alkyl, C₂- bis C₁₂-Alkenyl, C₄- bis C₇-Cycloalkyl, C₇- bis C₁₅-Aralkyl oder C₆- bis C₁₀-Aryl bedeuten,
- R³², R³³, R³⁶, R³⁷, R⁴⁰, R⁴¹, R⁴² bis R⁴⁵, R⁴⁷, R⁴⁸, R⁴⁹ bis R⁵² und R⁵⁵ bis R⁵⁸: unabhängig voneinander Wasserstoff, C₁- bis C₄-Alkyl, C₁- bis C₄-Alkoxy, Halogen, Cyan, Nitro, C₁- bis C₄-Alkoxycarbonyl, C₆- bis C₁₀-Aryl bedeuten
und
- R⁵⁷ und R⁵⁸: zusätzlich einen aromatischen oder quasiaromatischen fünf- oder sechsgliedrigen heterocyclischen Ring, der gegebenenfalls benzanneliert ist, bedeuten und R⁴⁸ zusätzlich NR⁷⁵R⁷⁶ bedeutet oder
- R⁴⁹ ; R⁵⁰ und/oder R⁵¹; R⁵²: eine -(CH₂)₃-, -(CH₂)₄-, -(CH₂)₅- oder -CH=CH-CH=CH- Brücke bilden,
- Z³: eine direkte Bindung, eine -CH=CH- oder -N=N-Brücke bedeutet,
- =Z⁴=: eine direkte Doppelbindung, eine =CH-CH= oder =N-N=-Brücke bedeutet,
- E³ bis E⁵, E¹⁰ und E¹¹: unabhängig voneinander O, S, NR⁵⁹ oder C(CH₃)₂ bedeuten und
- E⁵: zusätzlich C=O oder SO₂ bedeutet,
- E³ und E⁴: unabhängig voneinander zusätzlich -CH=CH- bedeuten können,
- E⁶ bis E⁹: unabhängig voneinander S, Se oder NR⁵⁹ bedeuten,
- R⁵⁹, R⁷⁵ und R⁷⁶: unabhängig voneinander C₁- bis C₁₂-Alkyl, C₂- bis C₈-Alkenyl, C₄- bis C₇-Cycloalkyl, C₇- bis C₁₅-Aralkyl, C₆- bis C₁₀-Aryl bedeuten.
und
- R⁷⁵: zusätzlich Wasserstoff bedeutet oder R⁷⁵ und R⁷⁶ in der Bedeutung von NR⁷⁵R⁷⁶ gemeinsam mit dem N-Atom, an das sie gebunden sind, einen fünf- oder sechsgliedrigen Ring bedeuten, der gegebenenfalls weitere Heteroatome enthält,
- R⁶¹ bis R⁶⁸: unabhängig voneinander Wasserstoff, C₁- bis C₆-Alkyl, C₁- bis C₄-Alkoxy, Cyan, C₁- bis C₄-Alkoxycarbonyl oder C₆- bis C₁₀-Aryl bedeuten und
- R⁶¹; R⁶² und R⁶⁷; R⁶⁸: unabhängig voneinander zusätzlich eine -(CH₂)₃-, -(CH₂)₄- oder -CH=CH-CH=CH-Brücke bilden oder
- R⁶² ; R⁶³, R⁶⁴; R⁶⁵ und R⁶⁶; R⁶⁷: eine -O-CH₂CH₂-O- oder -O-CH₂CH₂CH₂-O-Brücke bilden,
- v: eine ganze Zahl zwischen 0 und 10 000 bedeutet,
- R⁸², R⁸³, R⁸⁸ und R⁸⁹: unabhängig voneinander C₁- bis C₁₈-Alkyl, C₂- bis C₁₂-Alkenyl, C₄- bis C₇-Cycloalkyl, C₇- bis C₁₅-Aralkyl oder C₆- bis C₁₀-Aryl bedeuten,
- R⁸⁴ bis R⁸⁷ und R⁹⁰ bis R⁹³: unabhängig voneinander Wasserstoff oder C₁- bis C₆-Alkyl bedeuten oder
- R⁸⁴; R⁸⁶, R⁸⁵; R⁸⁷, R⁹⁰; R⁹² und/oder R⁹¹; R⁹³: gemeinsam eine -CH=CH-CH=CH- Brücke bilden.

Ebenfalls geeignet als RED₁ sind Anionen wie z.B. I⁻,I₃⁻, Br⁻, SCN⁻.

Über eine Brücke B verknüpfte, gegebenenfalls oligo- oder polymere Redoxsysteme sind beispielsweise solche der Formel

Y-[-(-B-Z-)ₐ-(-B-Y-)_{b}-]_{c}-B-Z (L),

worin
- Y und Z: unabhängig voneinander für einen Rest OX₂ oder RED₁ stehen, wobei aber mindestens ein Y für OX₂ und mindestens ein Z für RED₁ steht,
wobei
- OX₂: für den Rest eines reversibel elektrochemisch reduzierbaren Redoxsystems steht, und
- RED₁: für den Rest eines reversibel elektrochemisch oxidierbaren Redoxsystems steht,
- B: für ein Brückenglied steht,
- c: für eine ganze Zahl von 0 bis 1000 steht, und
- a und b: unabhängig voneinander für eine ganze Zahl von 0 bis 100 stehen.

Vorzugsweise ist (a+b)·c ≤ 10.000.

Hierbei ist unter reversibel elektrochemisch reduzierbar oder oxidierbar gemeint, daß die Elektronenübertragung ohne oder auch mit Änderung des σ-Gerüsts erfolgen kann ganz im Sinne der oben genannten Definition der erfindungsgemaßen OX₂ und RED₁.

Insbesondere sind mit den elektrochromen Verbindungen der Formel (L) solche der Formeln

OX₂-B-RED₁ (La),

OX₂-B-RED₁-B-OX₂ (Lb),

RED₁-B-OX₂-B-RED₁ (Lc),

oder

OX₂-(B-RED₁-B-OX_{2)d}B-RED₁ (Ld)

gemeint,
worin
- OX₂, RED₁ und B: die oben angegebene Bedeutung haben und
- d: für eine ganze Zahl von 1 bis 5 steht.

Mit OX₂ und RED₁ in den Formeln (L) und (La) bis (Ld) sind insbesondere Reste der oben beschriebenen Redoxsysteme der Formeln (I) bis (X), (CI) bis (CIV) und (XX) bis (XXXIII) gemeint, wobei die Bindung zum Brückenglied B über einen der Reste R² bis R¹⁹, R²² bis R²⁷, R²⁸ bis R⁵⁸, R⁶¹, R⁶², R⁶⁷, R⁶⁸, R⁸³, R⁸⁸, R¹²² oder im Falle, daß einer der Reste E¹ oder E² für NR¹ oder einer der Reste E³ bis E¹¹ für NR⁵⁹ oder einer der Reste E¹⁰¹ bis E¹⁰² für NR¹¹⁹ steht, über R¹, R⁵⁹ bzw. R¹¹⁹ erfolgt und die genannten Reste dann für eine direkte Bindung stehen, und
- B: für eine Brücke der Formeln -(CH₂)ₙ- oder - [Y¹ₛ(CH₂)ₘ-Y²]ₒ-(CH₂)ₚ-Y³_{q}- steht, die durch C₁- bis C₄-Alkyl, C₁- bis C₄-Alkoxy, Halogen oder Phenyl substituiert sein kann,
- Y¹ bis Y³: unabhängig voneinander für O, S, NR⁶⁰, COO, CONH, NHCONH, Cyclopentandiyl, Cyclohexandiyl, Phenylen oder Naphthylen stehen,
- R⁶⁰: C₁- bis C₆-Alkyl, C₂- bis C₆-Alkenyl, C₄- bis C₇-Cycloalkyl, C₇- bis C₁₅-Ar-alkyl oder C₆- bis C₁₀-Aryl bedeutet,
- n: eine ganze Zahl von 1 bis 12 bedeutet,
- m und p: unabhängig voneinander eine ganze Zahl von 0 bis 8 bedeuten,
- o: eine ganze Zahl von 0 bis 6 bedeutet und
- q und s: unabhängig voneinander 0 oder 1 bedeuten.

In ganz besonderem Maße sind mit OX, und RED, in den Formeln (L) und (La) bis (Ld) Reste der oben beschriebenen Redoxsysteme der Formeln (I), (V), (XX), (XXII), (XXIII), (XXV), (XXVI) und (XXXIII) gemeint.

Beispiele sind gemäß Formel (La) 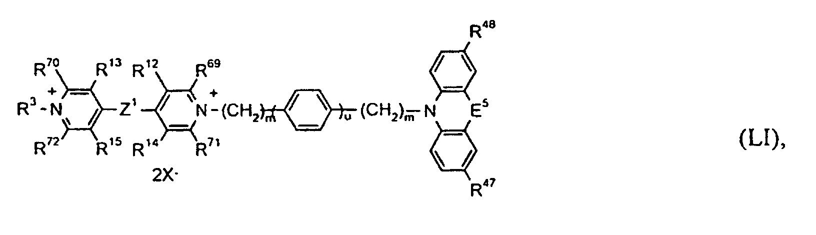 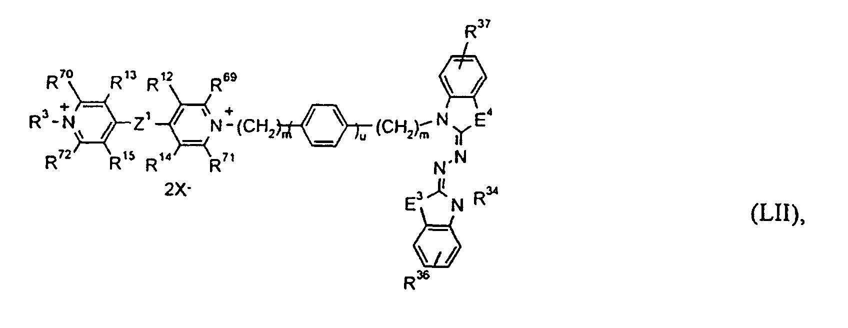 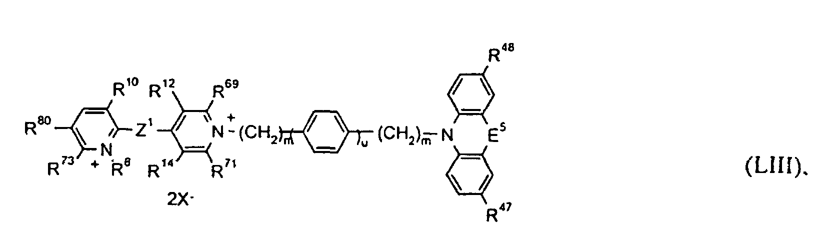 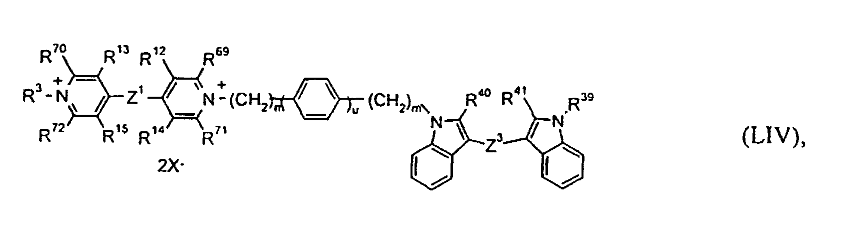 gemäß Formel (Lb) 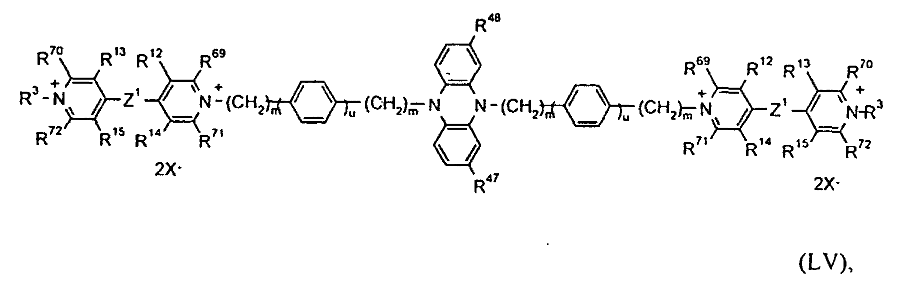 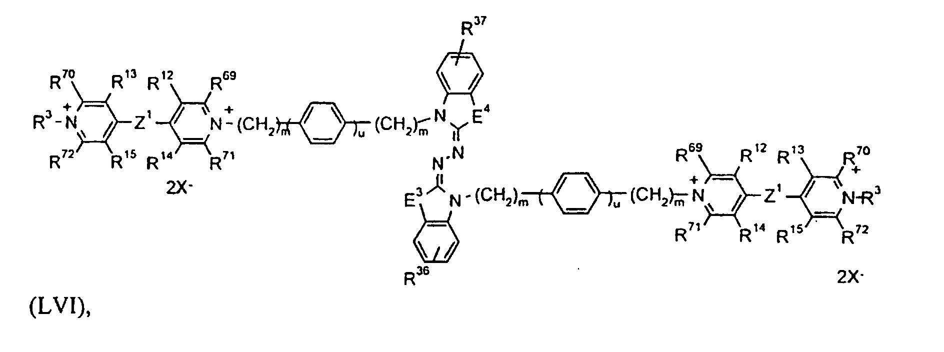 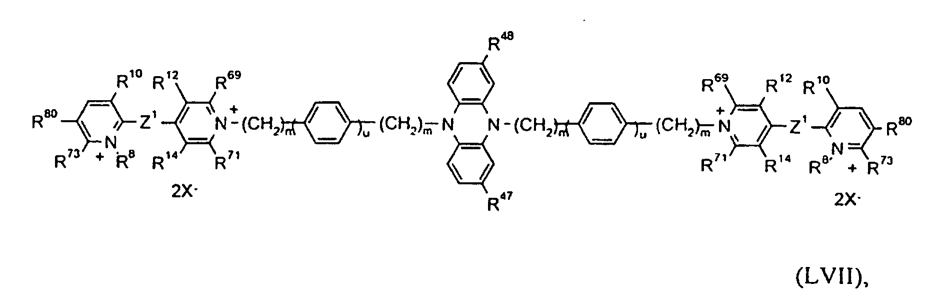 gemäß Formel (Lc) 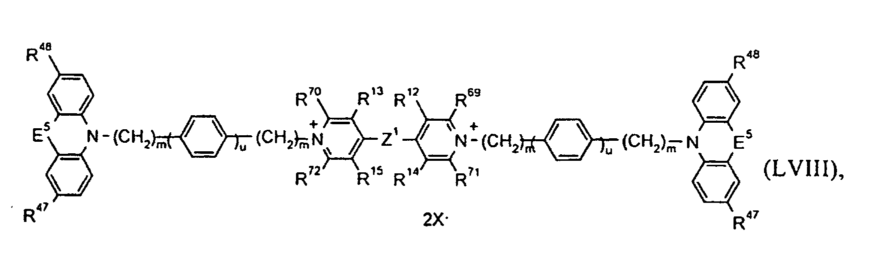 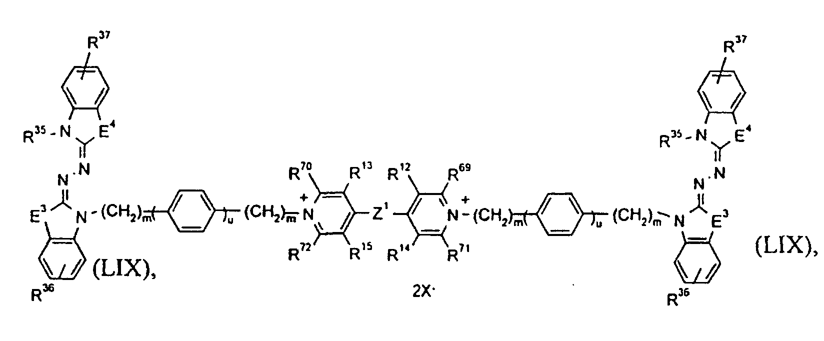 worin
- m: für eine ganze Zahl von 1 bis 5 steht,
- u: für 0 oder 1 steht und
die anderen Reste die oben angegebene Bedeutung besitzen.

In einem anderen Typ oligo- oder polymerer Systeme können die Gruppierungen OX₂ und/oder RED₁ auch beispielsweise als Seitenketten an einer Hauptgruppe, beispielsweise einem Poly(meth)acrylat, Silikon, Polycarbonat, Polyurethan, Polyharnstoff, Polyester, Polyamid, Cellulose oder anderen oligo- oder polymeren Systemen angebunden sein.

Beispiele für Metallsalze oder Metallkomplexe, die als OX, oder RED, eingesetzt werden können, sind Fe^{3+/2+}, Ni^{3+/2+}, Co^{3+/2+}, Cu^{2+/+}, [Fe(CN)₆]^{3-/4-}, Fe₄[Fe(CN)₆]₃^{0/4-}, [Co(CN)₆]^{3-/4-}, (Fe(Cyclopentadienyl)₂]^{0/4}, Lu(Pc)^{2+ bis 2-} (Pc = Phthalocyanin), Fe[Fe(CN)₆]^{0/4-}.

Als Gegenionen für Metallionen und kationische Komplexe kommen alle redox-inerten Anionen X⁻, wie sie später noch genauer beschrieben werden, in Frage, als Gegenionen der anionischen Komplexe alle redox-inerten Kationen M'⁺ in Frage, beispielsweise Alkalimetalle oder quaternierte Ammoniumsalze wie Na⁺, K⁺, N(CH₃)₄⁺, N(C₄H₉)₄⁺, C₆H₅CH₂N(CH₃)₃⁺ und andere.

Ebenfalls bevorzugt ist eine elektrochrome Vorrichtung, die Mischungen der oben allgemein und bevorzugt genannten elektrochromen Substanzen enthält. Beispiele für solche Mischungen sind (I) + (CI) + (XXVI), (I) + (IV) + (XXII), (La) + (I) + (XXVI), (La) + (CI), ohne daß dadurch irgendeine Einschränkung ausgedrückt werden soll.

Die Mischungsverhältnisse sind in weiten Grenzen variabel. Sie erlauben die Optimierung eines gewünschten Farbtons oder Schwärzegrades und/oder die Optimierung der gewünschten Dynamik der Vorrichtung.

In den oben genannten Substituentenbedeutungen sind Alkylreste, auch abgewandelte, beispielsweise Alkoxy- oder Aralkylreste, vorzugsweise solche mit 1 bis 12 C-Atomen, insbesondere mit 1 bis 8 C-Atomen, sofern nichts anderes angegeben ist. Sie können geradkettig oder verzweigt sein und gegebenenfalls weitere Substituenten tragen wie C₁- bis C₄-Alkoxy, Fluor, Chlor, Hydroxy, Cyano, C₁- bis C₄-Alkoxycarbonyl oder COOH.

Unter Cycloalkylresten werden vorzugsweise solche mit 3 bis 7 C-Atomen, insbesondere mit 5 oder 6 C-Atomen verstanden.

Alkenylreste sind vorzugsweise solche mit 2 bis 8 C-Atomen, insbesondere 2 bis 4 C-Atomen.

Arylreste, auch solche in Aralkylresten, sind Phenyl oder Naphthylreste, insbesondere Phenylreste. Sie können durch 1 bis 3 der folgenden Reste substituiert sein: C₁- bis C₆-Alkyl, C₁- bis C₆-Alkoxy, Fluor, Chlor, Brom, Cyano, Hydroxy, C₁- bis C₆-Alkoxycarbonyl oder Nitro. Zwei benachbarte Reste können auch einen Ring bilden.

Unter gegebenenfalls benzanellierten aromatischen oder quasiaromatischen fünfoder sechsgliedrigen heterocyclischen Ringen werden insbesondere Imidazol, Benzimidazol, Oxazol, Benzoxazol, Thiazol, Benzthiazol, Indol, Pyrazol, Triazol, Thiophen, Isothiazol, Benzisothiazol, 1,3,4- oder 1,2,4-Thiadiazol, Pyridin, Chinolin, Pyrimidin und Pyrazin verstanden. Sie können durch 1 bis 3 der folgenden Reste substituiert sein: C₁- bis C₆-Alkyl, C₁- bis C₆ Alkoxy, Fluor, Chlor, Brom, Cyano, Nitro, Hydroxy, Mono- oder Di-C₁- bis C₆-alkylamino, C₁- bis C₆-Alkoxycarbonyl, C₁- bis C₆-Alkylsulfonyl, C₁- bis C₆- Alkanoylamino, Phenyl oder Naphthyl. Zwei benachbarte Reste können auch einen Ring bilden.

Die elektrochromen Substanzen sind entweder bekannt (Topics in Current Chemistry, Vol. 92, S. 1-44, (1980), Angew. Chem. 90, 927 (1978), Adv. Mater. 3, 225, (1991), DE-OS 3.917.323, J. Am. Chem. Soc. 117, 8528 (1995), J. C. S. Perkin II 1990, 1777, DE-OS 4.435.211, EP-A 476.456, EP-A 476.457, DE-OS 4.007.058, J. Org. Chem. 57, 1849 (1992) und J. Am. Chem. Soc. 99, 6120, 6122 (1977)) oder lassen sich analog herstellen. Die Verbindungen der Formel (L) sind ebenfalls bekannt (WO 97/30134) oder lassen sich aus an sich bekannten Bausteinen beispielsweise nach folgendem Schema synthetisieren:

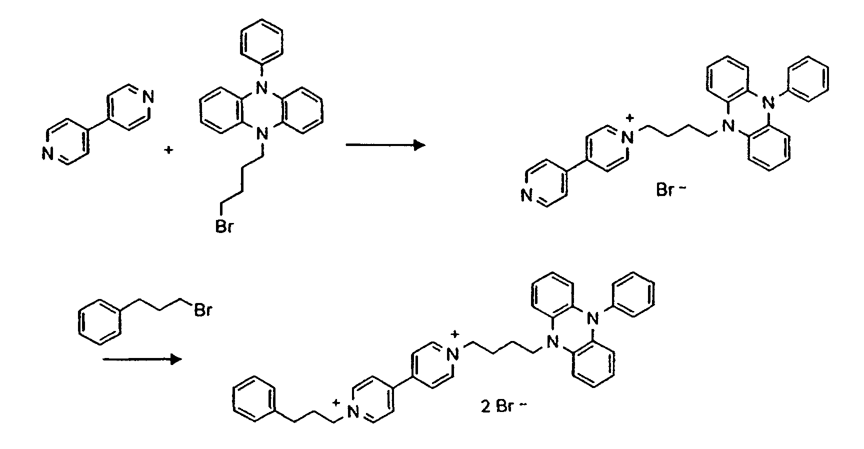

Synthetisch bedingte Ionen wie Bromid werden im Anschluß gegen redox-inerte Ionen ausgetauscht.

Besonders bevorzugt sind die elektrochromen Verbindungen der Formeln (I), (II), (III), (IV), (V), (XXII), (XXIII), (XXVI), (XXVII), (XXXI), (XXXII), (XXXIII), sowie die mindestens eine dieser Formeln als OX₂ bzw. RED₁ enthaltenden überbrückten Verbindungen der Formel (L).

Bei dieser Auswahl und ebenfalls bei den anschließend aufgeführten besonderen und herausragenden Auswahlen an elektrochromen Verbindungen muß stets sichergestellt sein, daß das elektrochrome Medium mindestens ein OX₂ und mindestens ein RED₁ enthält. Wenn beispielsweise OX₂ = Formel (I) ist, dann muß das elektrochrome Medium auch ein RED₁ enthalten, vorzugsweise aus der Auswahl der bevorzugten RED₁ der Formeln (XXII), (XXIII), (XXVI), (XXVII), (XXXI), (XXXII), und (XXXIII), aber auch aus der oben allgemein aufgeführten Auswahl der RED, der Formeln (XX) bis (XXXIII) sowie der oben erwähnten, als RED, geeigneten Metallsalze, -komplexe oder Anionen X⁻. Dies gilt analog auch für die bevorzugten und besonders bevorzugten RED₁.

Ganz besonders bevorzugt sind die elektrochromen Verbindungen der Formeln (I), (IV), (V), (XXII), (XXIII), (XXVII), (XXXIII),
worin
- R² R³, R⁸ und R⁹: unabhängig voneinander Methyl, Ethyl, Propyl, Butyl, Pentyl, Hexyl, Heptyl, Benzyl, Phenethyl, Phenylpropyl, Phenyl, 2-Methylphenyl oder 2,6-Dimethylphenyl bedeuten oder
- R⁸ und R⁹: gemeinsam eine -(CH₂)₂- oder -(CH₂)₃-Brücke bilden,
- R¹⁰ bis R¹⁵: Wasserstoff bedeuten,
- R⁶⁹ bis R⁷³, R⁸⁰ und R⁸¹: unabhängig voneinander Wasserstoff oder Methyl bedeuten oder
- R¹²; R⁶⁹, R¹³; R⁷⁰, R⁷³ ; R⁸⁰ und/oder R⁷⁴; R⁸¹: eine -CH=CH-CH=CH-Brücke bilden,
- Z': eine direkte Bindung oder -CH=CH- bedeutet,
- X⁻: ein unter den Bedingungen redox-inertes Anion bedeutet,
- R³⁴, R³⁵, R³⁸, R³⁹, R⁸⁸ und R⁸⁹: unabhängig voneinander Methyl, Ethyl, Propyl, Butyl, Benzyl, Phenethyl, Phenylpropyl oder Phenyl bedeuten,
- R³⁶ und R³⁷: Wasserstoff bedeuten,
- Z³: eine direkte Bindung oder eine -CH=CH-Brücke bedeutet,
- Z⁴: eine direkte Doppelbindung bedeutet,
- R⁴⁰ und R⁴¹: gleich sind und Wasserstoff oder Methyl bedeuten,
- E³ und E⁴: gleich sind und S, N-R⁵⁹ oder C(CH₃)₂ bedeuten,
- E⁶ bis E⁹: gleich sind und S bedeuten,
- R⁴⁹ bis R⁵²: unabhängig voneinander Wasserstoff, Methyl, Cyano oder Methoxycarbonyl bedeuten oder
- R⁴⁹; R⁵⁰ und/oder R⁵¹; R⁵²: eine -(CH₂)₃- oder -CH=CH-CH=CH-Brücke bilden,
- R⁹⁰ bis R⁹³: Wasserstoff bedeuten oder
- R⁹⁰; R⁹² und/oder R⁹¹; R⁹³: eine -CH=CH-CH=CH-Brücke bilden und
- R⁵⁹: Methyl, Ethyl, Propyl oder Butyl bedeutet,
sowie die mindestens eine dieser Formeln als OX₂ bzw. RED₁ enthaltenden überbrückten Verbindungen der Formel (L), insbesondere der Formel (La),
wobei
- B: -(CH₂)ₙ- bedeutet und
- n: eine ganze Zahl von 3 bis 6 bedeutet.

Im Sinne der Erfindung ganz herausragend geeignet sind die elektrochromen Verbindungen der Formel (I),
worin
- R² und R³: gleich sind und Methyl, Ethyl, Butyl, Heptyl oder Phenylpropyl bedeuten,
- R¹² bis R¹⁵ und R⁶⁹ bis R⁷²: Wasserstoff bedeuten,
- Z': eine direkte Bindung bedeutet und
- X⁻: ein redoxinertes Anion oder I⁻ bedeutet.

Im Sinne der Erfindung ebenfalls ganz herausragend geeignet sind die elektrochromen Verbindungen der Formel (La),
worin
- OX₂: für einen Rest der Formel (I) steht,
- RED₁: für einen Rest der Formel (XXVI) steht und
- B: für -(CH₂)ₙ- steht,
wobei
- n: eine ganze Zahl von 3 bis 6 bedeutet,
- R² und R⁴⁶: eine direkte Bindung zu B bedeuten,
- R³, R¹² bis R¹⁵, R⁶⁹ bis R⁷², Z¹ und X⁻: die oben angegebene herausragende Bedeutung besitzen,
- R⁴⁷ und R⁴⁸: Wasserstoff bedeuten,
- E⁵: NR⁵⁹ bedeutet und
- R⁵⁹: Methyl, Ethyl, Butyl, Heptyl, Phenylpropyl oder Phenyl bedeutet.

Die erfindungsgemäße lichtgeschützte elektrochrome Vorrichtung enthält in ihrem elektrochromen Medium vorzugsweise mindestens ein Lösungsmittel, in dem die elektrochromen Substanzen, gegebenenfalls ein Leitsalz und gegebenenfalls weitere Zusätze gelöst sind. Das Lösungsmittel kann auch gelförmig verdickt sein, beispielsweise durch Polyelektrolyte, poröse Feststoffe oder Nanopartikel mit großer aktiver Oberfläche.

Geeignete Lösungsmittel sind alle unter den gewählten Spannungen redox-inerten Lösungsmittel, die keine Elektrophile oder Nukleophile abspalten können oder selber als ausreichend starke Elektrophile oder Nukleophile reagieren und so mit den farbigen Radikalionen reagieren könnten. Beispiele sind Propylencarbonat, γ-Butyrolacton, Acetonitril, Propionitril, Benzonitril, Glutaronitril, Methylglutamitril, 3,3'-Oxydipropionitril, Hydroxypropionitril, Dimethylformamid, N-Methylpyrrolidon, Sulfolan, 3-Methylsulfolan oder Mischungen davon. Bevorzugt sind Propylencarbonat, Benzonitril und Mischungen untereinander oder mit Glutaronitril oder 3-Methylsulfolan. Insbesondere bevorzugt ist Propylencarbonat. Ebenfalls insbesondere bevorzugt ist Benzonitril.

Die elektrochrome Lösung kann mindestens ein inertes Leitsalz enthalten. Insbesondere wenn wenigstens eine der Substanzen des Redoxpaares RED₁/OX₂ ionischer Natur ist, kann auf den Zusatz eines Leitsalzes verzichtet werden.

Als inertes Leitsalz sind Lithium-, Natrium- und Tetraalkylammoniumsalze geeignet, insbesondere letztere. Die Alkylgruppen können zwischen 1 und 18 C-Atome aufweisen und gleich oder verschieden sein. Bevorzugt ist Tetrabutylammonium. Als Anionen zu diesen Salzen, aber auch als Anionen X⁻ in den Formeln (I) bis (VI), (CI), (CII) und (CV) bis (CVII) und in den Metallsalzen kommen alle redox-inerten, farblosen Anionen in Frage.

Beispiele sind Tetrafluoroborat, Tetraphenylborat, Cyano-triphenylborat, Tetramethoxyborat, Tetrapropoxyborat, Tetraphenoxyborat, Perchlorat, Chlorid, Nitrat, Sulfat, Phosphat, Methansulfonat, Ethansulfonat, Tetradecansulfonat, Pentadecansulfonat, Tritluormethansulfonat, Perfluorbutansulfonat, Perfluoroctansulfonat, Benzolsulfonat, Chlorbenzolsulfonat, Toluolsulfonat, Butylbenzolsulfonat, tert. Butylbenzolsulfonat, Dodecylbenzolsulfonat, Trifluormethylbenzolsulfonat, Hexafluorophosphat, Hexafluoroarsenat, Hexafluorosilicat, 7,8- oder 7,9-Dicarbanidoundecaborat(-1) oder (-2), die gegebenenfalls an den B- und/oder C-Atomen durch eine oder zwei Methyl-, Ethyl-, Butyl- oder Phenyl-Gruppen substituiert sind, Dodecahydro-dicarbadodecaborat(-2) oder B-Methyl-C-phenyl-dodecahydro-dicarbadodecaborat(-1).

Ebenfalls geeignet, auch als Anionen X⁻ in den Formeln (I) bis (VI), (CI), (CII) und (CV) bis (CVII) und in den Metallsalzen, sind die oben erwähnten Anionen, die auch die Rolle eines RED₁ übernehmen können, beispielsweise I⁻,I₃⁻.

Die Leitsalze werden vorzugsweise im Bereich 0 bis 1 mol/l eingesetzt.

Als weitere Zusätze können Verdicker eingesetzt werden, um die Viskosität der elektroaktiven Lösung zu steuern. Das kann Bedeutung haben zur Vermeidung von Segretation, d.h. der Bildung von streifiger oder fleckiger Farbbildung bei längerem Betrieb der elektrochromen Vorrichtung im eingeschalteten Zustand, und zur Steuerung der Ausbleichgeschwindigkeit nach Abschalten des Stroms.

Als Verdicker eignen sich alle für diesen Zweck üblichen Verbindungen wie z. B. Polyacrylat, Polymethacrylat (Luctite L®), Polycarbonat oder Polyurethan.

Als weitere Zusätze für die elektrochrome Lösung kommen zum fallweise erwünschten Schutz vor UV-Licht (< 350 nm) UV-Absorber in Frage. Beispiele sind UVINUL® 3000 (2,4-Dihydroxybenzophenon, BASF), SANDUVOR® 3035 (2-Hydroxy-4-n-octyloxybenzophenon, Clariant), Tinuvin® 571 (2-(2H-Benzotriazol-2-yl)-6-dodecyl-4-methylphenol, Ciba), Cyasorb 24™ (2,2'-Dihydroxy-4-methoxybenzophenon, American Cyanamid Company), UVINUL® 3035 (Ethyl-2-cyano-3,3-diphenylacrylat, BASF), UVINUL® 3039 (2-Ethylhexyl-2-cyano-3,3-diphenylacrylat, BASF), UVINUL® 3088 (2-Ethyihexyl-p-methoxycinnamat, BASF), CHIMASSORB® 90 (2-Hydroxy-4-methoxy-benzophenon, Ciba).

Bevorzugt sind die vier Letztgenannten. Ebenfalls bevorzugt sind Mischungen von UV-Absorbern, beispielsweise der vier Letztgenannten. Besonders bevorzugt ist die Mischung aus UVINUL® 3039 und CHIMASSORB® 90.

Die UV-Absorber werden im Bereich 0.01 bis 2 mol/l, vorzugsweise 0,04 bis 1 mol/l eingesetzt.

Die elektrochrome Lösung enthält die elektrochromen Substanzen OX₂ und RED₁, insbesondere die der Formeln (I) bis (X), (XX) bis (XXXIII), (CI) bis (CIV) und (L) jeweils in einer Konzentration von mindestens 10⁻⁴ mol/l, vorzugsweise 0,001 bis 0,5mol/l. Die Gesamtkonzentration aller enthaltenen elektrochromen Substanzen liegt vorzugsweise unter 1 mol/l.

Die erfindungsgemäßen Gelbfilter, insbesondere die Gelbfarbstoffe der Formeln (CCI) bis (CCVIII), können ebenfalls in dem elektrochromen Medium gelöst sein. Sie werden im Konzentrationsbereich von 0,01 bis 2 mol/l, vorzugsweise von 0.04 bis 1 mol/l eingesetzt.

Sie können aber auch in den Platten oder Folien gelöst oder fein dispergiert sein, wenn es sich um Platten oder Folien aus Kunststoffmaterial handelt. Geeignete Materialien sind Polycarbonat, Polymethylmethacrylat, Polyester, Polyolefin, Polyacetat oder Zellulose-Derivate. Die Gelbfarbstoffe der Formeln (CCI) bis (CCVIII) und die Fluoreszenzfarbstoffe der Formeln (CCIX) bis (CCXIII) werden im Konzentrationsbereich von 0,01 Gew.-% bis 30 Gew.-% eingesetzt, bevorzugt sind 0, 1 bis 20 Gew.-%.

Sie können aber auch in einer Beschichtung dieser Platten oder Folien gelöst oder fein dispergiert sein. Diese Anwendungsform ist besonders bevorzugt. Diese Beschichtungen sind in der Regel auf der Außenseite der elektrochromen Vorrichtung angebracht, d. h. auf der Seite der Platten oder Folien, die nicht leitfähig beschichtet sind. Geeignete Beschichtungsmaterialien sind alle transparenten, auf den Platten oder Folien gut haftenden Materialien, wie Kunststoffe oder Lacke, beispielsweise Polyurethane, Polyvinylalkohol, Polyvinylacetat, Polyacrylate, Polymethacrylate, Polyester, Polyamid, Polyacrylnitril, oder entsprechende Misch- oder Copolymerisate, usw. Diese Beschichtungsmaterialien können jedoch auch Folien sein, die mittels Klebern auf die Platten oder Folien aufgebracht werden. Die Beschichtungsmaterialien haben eine Dicke von 0,1 bis 500 µm. Die Gelbfarbstoffe der Formeln (CCI) bis (CCXIII) werden im Konzentrationsbereich von 0,01 Gew.-% bis 30 Gew.-% in den Beschichtungsmaterialien eingesetzt.

Die Gelbfarbstoffe und/oder Fluoreszenzfarbstoffe können in der elektrochromen Lösung und/oder in den Platten oder Folien und/oder in den Beschichtungsmaterialien eingesetzt werden. Sie können auch untereinander gemischt werden bzw. es können in den verschiedenen Anwendungsformen verschiedene Gelbfarstoffe und/oder Fluoreszenzfarbstoffe eingesetzt werden.

Die Nanoteilchen werden vorzugsweise in den Platten oder Folien und/oder in Beschichtungsmaterialien dispergiert eingesetzt. Sie können aber auch in dem elektrochromen System dispergiert sein.

Bei der Anwendung in den Platten oder Folien sollen diese aus Kunststoffmaterial sein. Geeignete Materialien sind oben beschrieben. Die Nanoteilchen werden im Konzentrationsbereich von 0,001 bis 30, vorzugsweise 0,01 bis 10 Atom-% eingesetzt.

Bei der Anwendung in Beschichtungsmaterialien, die besonders bevorzugt ist, werden Materialien verwendet, die bereits oben aufgeführt sind. Die Nanoteilchen werden im Konzentrationsbereich von 0,001 bis 30, vorzugsweise 0,01 bis 10 Atom-% eingesetzt.

Die Einarbeitung der Nanoteilchen in solche Materialien kann nach gängigen Methoden erfolgen, beispielsweise gemäß WO-A 95/09 895, WO-A 92/21 315, EP-A 0 628 303.

Zusätzlich können in den Platten oder Folien und/oder Beschichtungsmaterialien auch UV-Absorber enthalten sein, wie sie weiter oben beschrieben sind. Sie dienen hier vorzugsweise zum Lichtschutz der Platten-, Folien- oder Beschichtungsmaterialien sowie zum UV-Schutz der erfindungsgemäßen Gelbfarbstoffe.

Es können aber auch, eventuell zusätzlich, andere Lichtschutzmaterialien wie Quencher oder Radikalfänger verwendet werden, wie sie Kunststoffbereich üblich sind, beispielsweise UVINUL® 4049H (BASF), UVINUL® 4050H (BASF).

Zum Betrieb der erfindungsgemäßen elektrochromen Vorrichtung wird ein konstanter, gepulster oder in seiner Amplitude sich verändernder, beispielsweise sich sinusförmig verändernder, Gleichstrom benutzt. Die Spannung hängt ab von der gewünschten Farbtiefe, insbesondere aber von den Reduktions- bzw. Oxidationspotentialen der verwendeten OX₂ und RED₁. Solche Potentiale können beispielsweise aus Topics in Current Chemistry, Volume 92, S. 1-44, (1980) oder Angew. Chem. 90, 927 (1978) oder der dort zitierten Literatur entnommen werden. Die Differenz ihrer Potentiale ist ein Richtwert für die erforderliche Spannung, jedoch kann die elektrochrome Vorrichtung bereits bei niedrigerer oder auch mit höherer Spannung betrieben werden. In vielen Fällen, z. B. bei Verwendung von OX₂ = Formel (I) oder (V) und RED₁ = Formel (XX), (XXII), (XXVI) oder (XXVII) oder deren Verknüpfung über eine Brücke gemäß Formel (L), insbesondere Formel (La) bis (Ld), liegt diese zum Betrieb nötige Potentialdifferenz ≤ 1 V. Solche elektrochromen Vorrichtungen können deshalb in einfacher Weise mit dem Strom aus photovoltaischen Siliciumzellen versorgt werden.

Wird die Spannung abgeschaltet, geht die erfindungsgemäße elektrochrome Vorrichtung wieder in ihren ursprünglichen Zustand zurück. Diese Löschung kann erheblich beschleunigt werden, wenn die kontaktierten Segmente bzw. Platten kurzgeschlossen werden. Auch durch mehrmaliges Umpolen der Spannung, gegebenenfalls auch bei gleichzeitiger Erniedrigung der Spannung, kann die Anzeige sehr rasch gelöscht werden.

Durch Variation der Schichtdicke der elektrochromen Vorrichtung, der Viskosität der elektrochromen Lösung und/oder der Diffusions- oder Driftfähigkeit der elektrochromen Substanzen lassen sich die Einschalt- und Ausschaltzeiten der Anzeigevorrichtung in weiten Grenzen beeinflussen. So zeigen beispielsweise dünne Schichten kürzere Schaltzeiten als dicke. Es lassen sich also schnell und langsam schaltbare Vorrichtungen bauen und so den jeweiligen Einsatzzwecken optimal anpassen.

Bei langsamen Vorrichtungen, insbesondere Anzeigevorrichtungen, kann zur Aufrechterhaltung der angezeigten Information im eingeschalteten Zustand ein Stromspar- oder Refresh-Mode benutzt werden. Nach Aufbau der anzuzeigenden Information beispielsweise durch konstante oder sich mit hoher Frequenz verändernder oder gepulster Gleichspannung ausreichender Höhe wird auf gepulste oder sich verändernde Gleichspannung niedriger Frequenz umgeschaltet, wobei während der Phasen, in denen die Spannung Null beträgt, die Kontaktierung der Segmente nicht kurzgeschlossen wird. Diese niedrige Frequenz kann beispielsweise im Bereich von 1 Hz oder niedriger liegen, wobei die Dauer der Einschalt- und Ausschaltphasen nicht gleichlang zu sein brauchen, sondern beispielsweise die Ausschaltphasen deutlich länger sein können. Da sich während der Strompausen im nicht kurzgeschlossenen Zustand die Farbtiefe der angezeigten Information nur langsam abbaut, genügen relativ kurz Stromimpulse, um diese Verluste in der anschließenden Refresh-Phase wieder auszugleichen. Man erhält so ein flackerfreies Bild mit nahezu konstanter Farbtiefe, für dessen Aufrechterhaltung aber nur ein Bruchteil des Stromes benötigt wird, der bei permanentem Stromfluß anfallen würde.

Spezielle Ausführungsformen der obengenannten Typen 1 und 2 können beispielsweise die folgenden sein, die ebenfalls Gegenstand der Erfindung sind, wenn sie durch Gelbfilter vor Licht geschützt sind.

### Typ 1: (unverspiegelt)

aus dem Bereich Lichtschutz/Lichtfilter: Fensterscheiben für Gebäude, Straßenfahrzeuge, Flugzeuge, Eisenbahnen, Schiffe, Dachverglasungen, Autosonnendächer, Verglasung von Gewächshäusern und Wintergärten, Lichtfilter beliebiger Art;
aus dem Bereich Sicherheit/Geheimhaltung: Trennscheiben für Raumteiler, beispielsweise in Büros, Straßenfahrzeugen, Flugzeugen, Eisenbahnen, Sichtschutzscheiben, beispielsweise an Bankschaltern, Türverglasungen, Scheiben für Motorrad- oder Pilotenhelme;
aus dem Bereich Design: Verglasung von Backöfen, Mikrowellengeräten, anderen Haushaltsgeräten, Möbeln.

Aus dem Bereich Anzeigen: analoge Spannungsanzeigen, als Batterietester, Tankanzeigen, Temperaturanzeigen, usw.

### Typ 1: (verspiegelt)

Spiegel jeglicher Art, beispielsweise für Straßenfahrzeuge, Eisenbahnen, insbesondere plane, spärische, asphärische Spiegel und Kombinationen daraus, beispielsweise spärisch/asphärisch, Spiegelverglasung in Möbeln.

### Typ 2:

Anzeigevorrichtungen jeglicher Art, beispielsweisse Segment- oder Matrixanzeigen, beispielsweise für Uhren, Computer, Elektrogeräte, Elektronikgeräte wie Radios, Verstärker, Fernscher, CD-Player, Zielanzeige in Bussen und Zügen, Abfahrts- oder Abfluganzeigen in Bahnhöfen und Flughäfen, Flachbildschirme, alle Anwendungen, die unter Typ 1 und 2 genannt sind, die mindestens eine schaltbare, statische oder variable Anzeigevorrichtung enthalten, beispielsweise Trennscheiben, die Anzeigen wie "Bitte nicht stören", "Schalter nicht besetzt" enthalten, beispielsweise Auto-Spiegel, die Anzeigen beliebiger Art enthalten, wie Anzeige der Temperatur, Störungen im Fahrzeug, beispielsweise Öltemperatur, offene Türen, Zeit, Himmelsrichtung.

Ein weiterer Gegenstand der Erfindung sind elektrochrome Substanzen der Formel
worin
- R³: -(CH₂)₃-C₆H₅ oder -(CH₂)₄-C₆H₅ bedeutet,
- Z¹: eine direkte Bindung bedeutet,
- E⁵: N-C₆H₅, N-(CH₂)₃-C₆H₅ oder N-(CH₂)₄-C₆H₅ bedeutet,
- m: eine ganze Zahl von 3 bis 5 bedeutet und
- X⁻: ein unter den Bedingungen redox-inertes Anion bedeutet.

Besonders bevorzugt sind elektrochrome Substanzen der Formel (LX),
worin
- R³: -(CH₂)₃-C₆H₅ bedeutet,
- Z¹: eine direkte Bindung bedeutet,
- E⁵: N-C₆H₅ bedeutet,
- m: eine ganze Zahl von 3 bis 5 bedeutet und
- X⁻: ein unter den Bedingungen redox-inertes Anion bedeutet.

Ganz besonders bevorzugt sind elektrochrome Substanzen der Formel (LX),
worin
- R³: -(CH₂)₃-C₆H₅ bedeutet,
- Z¹: eine direkte Bindung bedeutet,
- E⁵: N-C₆H₅ bedeutet,
- m: eine ganze Zahl von 4 bedeutet und
- X⁻: BF₄ bedeutet.

Diese erfindungsgemäßen elektrochromen Substanzen der Formel (LX) zeichnen sich selbst durch eine höhere Lichtechthcit aus verliehen mit ähnlichen bekannten Verbindungen, z. B. solchen der Formel (LX), worin R³ Benzyl bedeutet.

### Beispiele

### Beispiel 1

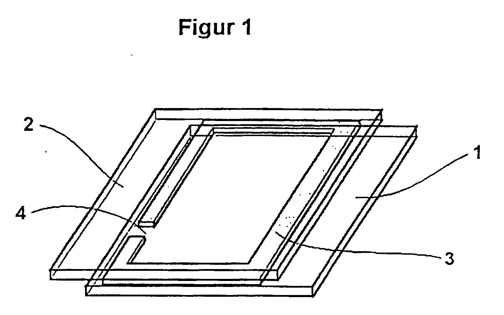

Gemäß Fig. 1 wurde eine Zelle aufgebaut. Es wurden hierzu zwei Glasplatten 1 und 2 benutzt, die auf einer Fläche mit ITO beschichtet sind.

Eine Mischung aus 97 % photohärtendem Epoxikleber DELO-Katiobond® 4594 (DELO Industrieklebstoffe, Landsberg) und 3 % Glaskugeln mit 200 µm Durchmesser wurde ringförmig 3 auf die mit ITO-beschichtete Seite der Glasplatte 1 so aufgetragen, daß eine 2 mm breite Öffnung 4 ausgespart wurde. Nun wurde die Glasplatte 2 so auf die Kleberaupe gelegt, daß die ITO-Schichten der beiden Platten 1 und 2 einander zugewandt waren und eine Geometrie entstand, wie in Fig. 1 gezeigt. Die Aushärtung des Klebers erfolgte durch 10-minütiges Belichten mit Tageslicht in der Nähe eines Fensters und anschließend für 20 min bei 105°C ohne Belichtung.

Eine Schale wurde unter Stickstoffatmosphäre mit einer Lösung gefüllt, die 0,02 molar an der elektrochromen Verbindung der Formel 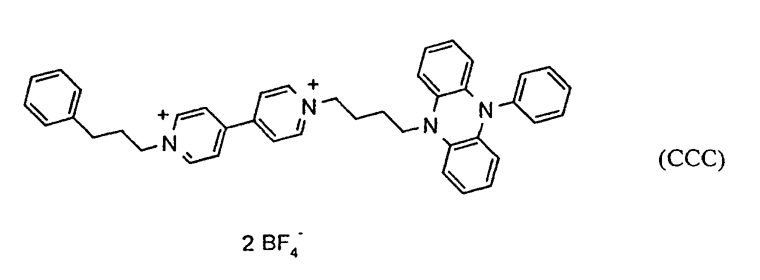 und jeweils 0,1 molar an den UV-Absorbern der Formeln 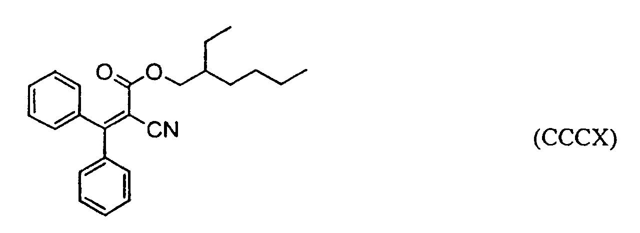 und 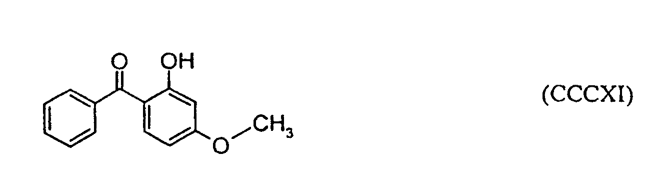 in wasserfreiem, sauerstofffreiem Propylencarbonat war und 2 Gew.-% des Gelbfilters der Formel 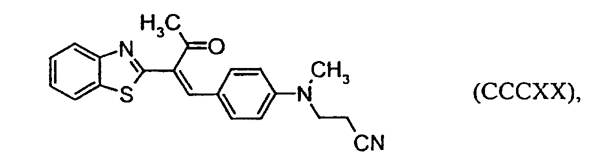 mit λₘₐₓ = 383 nm enthielt.

Dann wurde die Zelle unter Stickstoffatmosphärc senkrecht so in die Schale gestellt, daß die Öffnung 4 sich unterhalb des Flüssigkeitsspiegels befand. Die Schale mit der Zelle wurde in einen Exsiccator gestellt. Dieser wurde auf 0,05 mbar evakuiert und anschließend vorsichtig mit Stickstoff belüftet. Während der Belüftung stieg die elektrochrome Lösung durch die Öffnung 4 in die Zelle hinein und füllte bis auf eine kleine Blase das gesamte Volumen aus. Die Zelle wurde aus der Lösung entnommen, unter Stickstoffatmosphäre an der Öffnung 4 gereinigt, indem sie mit einem Papiertuch abgeputzt wurde, und mit dem photochemisch härtbaren Acrylatkleber DELO-Photobond®4497 (DELO Industrieklebstoffe, Landsberg) verschlossen. Anschließend wurde 1 min unter Stickstoffatmosphäre mit der Lampe DELOLUX®03 (DELO Industrieklebstoffe, Landsberg) die sich in einem Abstand von 8 cm zur Öffnung 4 befand, belichtet und bei Raumtemperatur über Nacht unter Stickstoffatmosphäre ausgehärtet.

Durch Anlegen einer Spannung von 0,9 V an die beiden Platten 1 und 2 färbte sich die Zelle rasch tief grünlich blau. Durch Abschalten der Spannung und Kurzschließen der Kontakte verschwand die Färbung wieder rasch.

### Beispiel 1a (Vergleich)

Es wurde eine Zelle gebaut wie in Beispiel 1 beschrieben, jedoch ohne den Gelbfilter der Formel (CCCXX).

### Lichtstabilitätstest

Zum Test der Lichtstabilität wurden Zellen gemäß Beispiel 1 gemeinsam mit Referenzzellen gemäß Beispiel 1a bei einer Betriebsspannung von 0,9 V in einem Testgerät Suntest CPS+ der Firma Atlas, Linsengericht-Altenhaßlau ausgerüstet mit der Filterschale A und einer Bestrahlungsleistung von 765 W/m² belichtet.

Vor Beginn der Bestrahlung wurden mit einem Absorptionsphotometer Cary 4G (Firma Varian, Darmstadt) Absorptionsspektren jeder Zelle im geschalteten (0,9 V) und im ungeschalteten Zustand (0 V) aufgenommen.

Die Bestrahlung erfolgte in sich jeweils verdoppelnden Intervallen (30 Minuten, 1 Stunde, 2 Stunden, 4 Stunden, 8 Stunden, 16 Stunden), so daß jede Probe am Ende insgesamt 31,5 Stunden bestrahlt worden war. Nach jedem Bestrahlungsintervall wurden wieder Absorptionsmessungen in geschalteten und ungeschalteten Zustand durchgeführt. Aus diesen Messungen wurden die Differenzspektren gebildet, wobei jeweils im geschalteten und ungeschalteten Zustand die aktuellen Spektren minus der Ausgangsspektren aufgetragen werden.

Die Schädigung der Zelle wurde durch die Abnahme des elektrochromen Hubes definiert. Dies bedeutet die Abnahme der Transmissionsänderung bei einer bestimmten Wellenlänge.

Ausgewertet wurde die Maximumswellenlänge bei 605nm. Bei der Auswertung der Differenzspektren muß beachtet werden, daß Änderungen der Transmission im ungeschalteten Zustand auch in den Differenzspektren des geschalteten Zustands auftauchen und dort substrahiert werden müssen.

In der folgenden Tabelle sind die Abnahme des elektrochromen Hubs über die kumulierte Bestrahlungszeit für die erfindungsgemäßc Zelle mit dem Gelbfilter (gemäß Beispiel 1) und als Vergleich dazu die ohne Gelbfilter (gemäß Beispiel 1a) aufgetragen.

| In % | 0h | 0,5h | 1,5h | 3,5h | 7,5h | 15,5h | 31,5h |
|---|---|---|---|---|---|---|---|
| Zelle mit Gelbfilter | 100 | 100 | 100 | 100 | 100 | 98 | 60 |
| Zelle ohne Gelbfilter | 100 | 70 | 50 | 43 | 35 | - | - |

Definiert man eine signifikante Schädigung der Zelle aus Verlust von 20% des elektrochromen Hubs, so ergibt sich ohne Gelbfilter eine Stabilität von 19 Minuten. Mit Gelbfilter erhöht sich diese Stabilität auf 25 Stunden, dies entspricht einer Verbesserung um einen Faktor 80.

Ganz analog wurden als elektrochrome Substanzen und als Gelbfilter die Verbindungen der Beispiele 1-3 bis 1-15 eingesetzt:

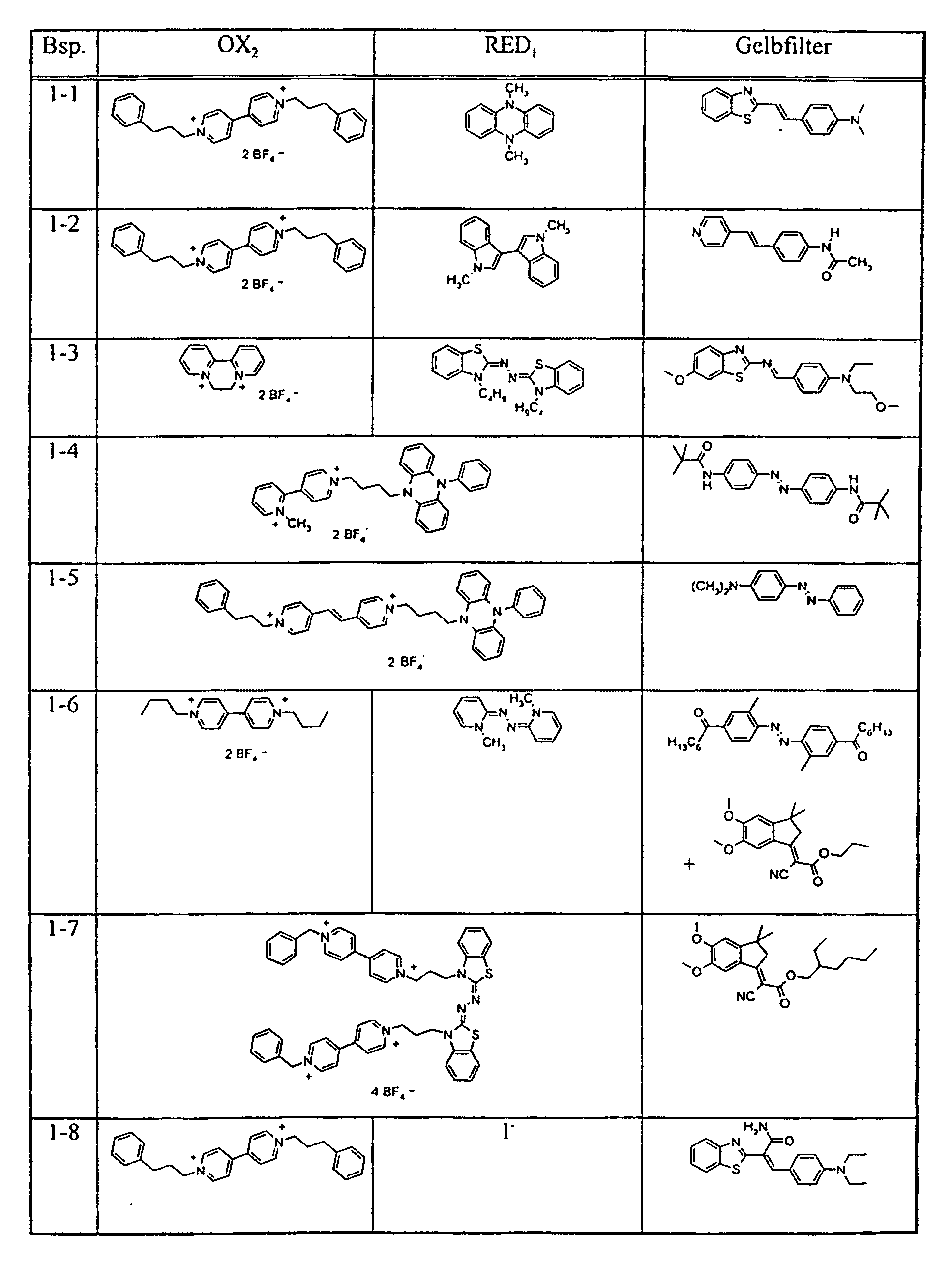

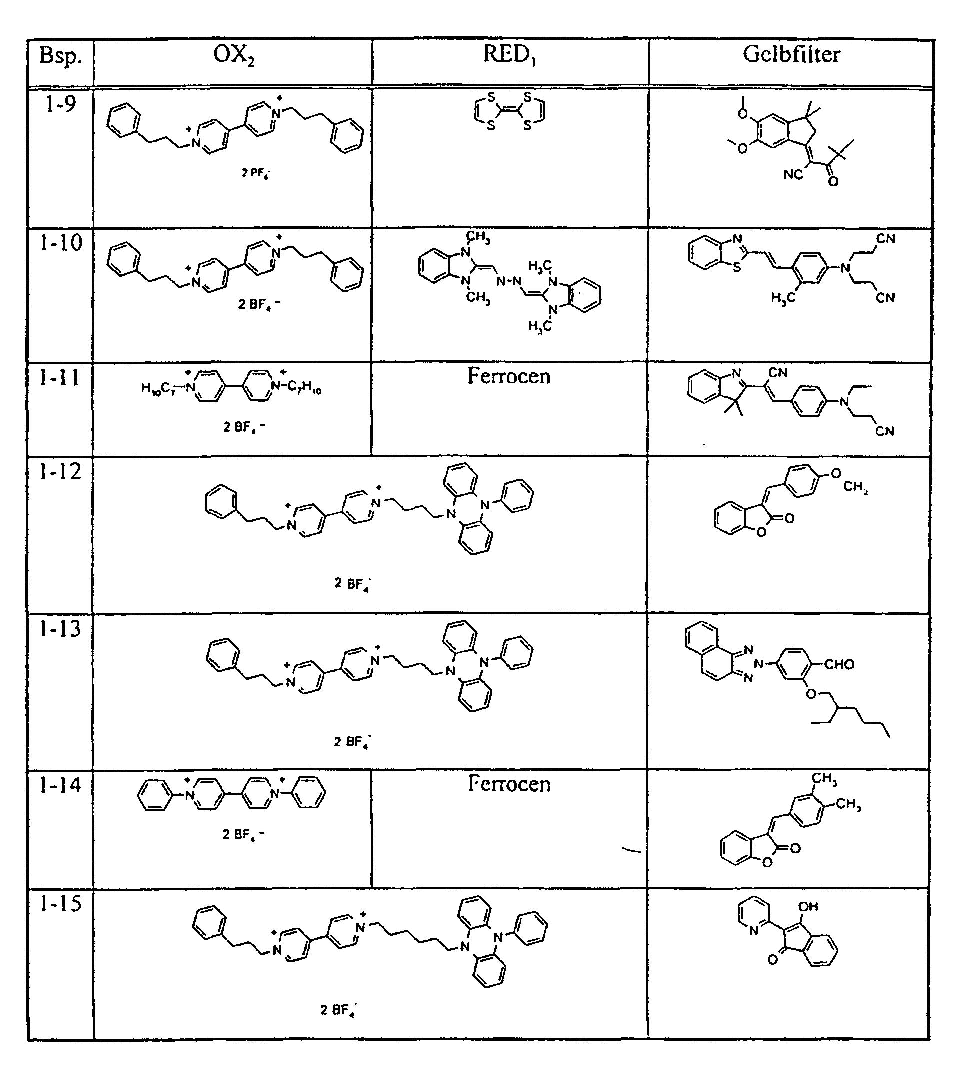

### Beispiel 2

Es wurden wie in Beipiel 1 zwei Platten verwendet, die auf einer Seite mit ITO beschichtet sind. Auf der anderen Seite wurde ein PU-Lack aus Desmodur und Desmophen (Bayer AG), der 0,9 Gewichtsprozent des Gelbfilters der Formel 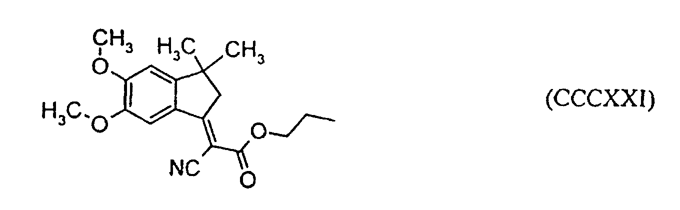 enthielt, durch Rakeln in einer Naßfilmdicke von 240 µm aufgebracht. Die Filme wurden über Nacht bei 130°C getrocknet.
Aus diesen Platten wird analog Beispiel 1 eine Zelle gebaut.

Die Zelle wurde mit einer Lösung gefüllt. die 0,02 molar an der elcktrochromen Verbindung der Formel (CCC) (s. Beispiel 1) in wasserfreiem, sauerstofffreiem Propylencarbonat war. Die Zelle wurde wie in Beispiel 1 beschrieben verschlossen.

### Beispiel 2a (Vergleich)

Es wurde eine Zelle gebaut wie in Beispiel 2, wobei jedoch Glasplatten verwendet wurden. die nur mit ITO beschichtet waren.

### Lichtstabilitätstest

Die Zellen der Beispiele 2 (erfindungsgemäß) und 2a wurden, wie oben beschrieben, unter den gleichen Testbedingungen auf Lichtstabilität geprüft. Die Zelle gemäß Beispiel 2 war lichtechter als die Zelle gemäß Beispiel 2a.

Ganz analog wurden als elektrochrome Substanzen und als Gelbfilter die Verbindungen der Beispiele 2-1 bis 2-12 eingesetzt:

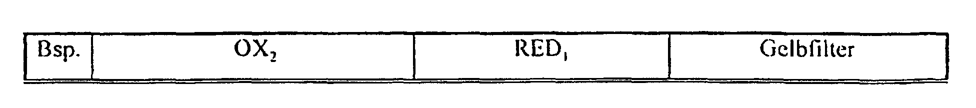

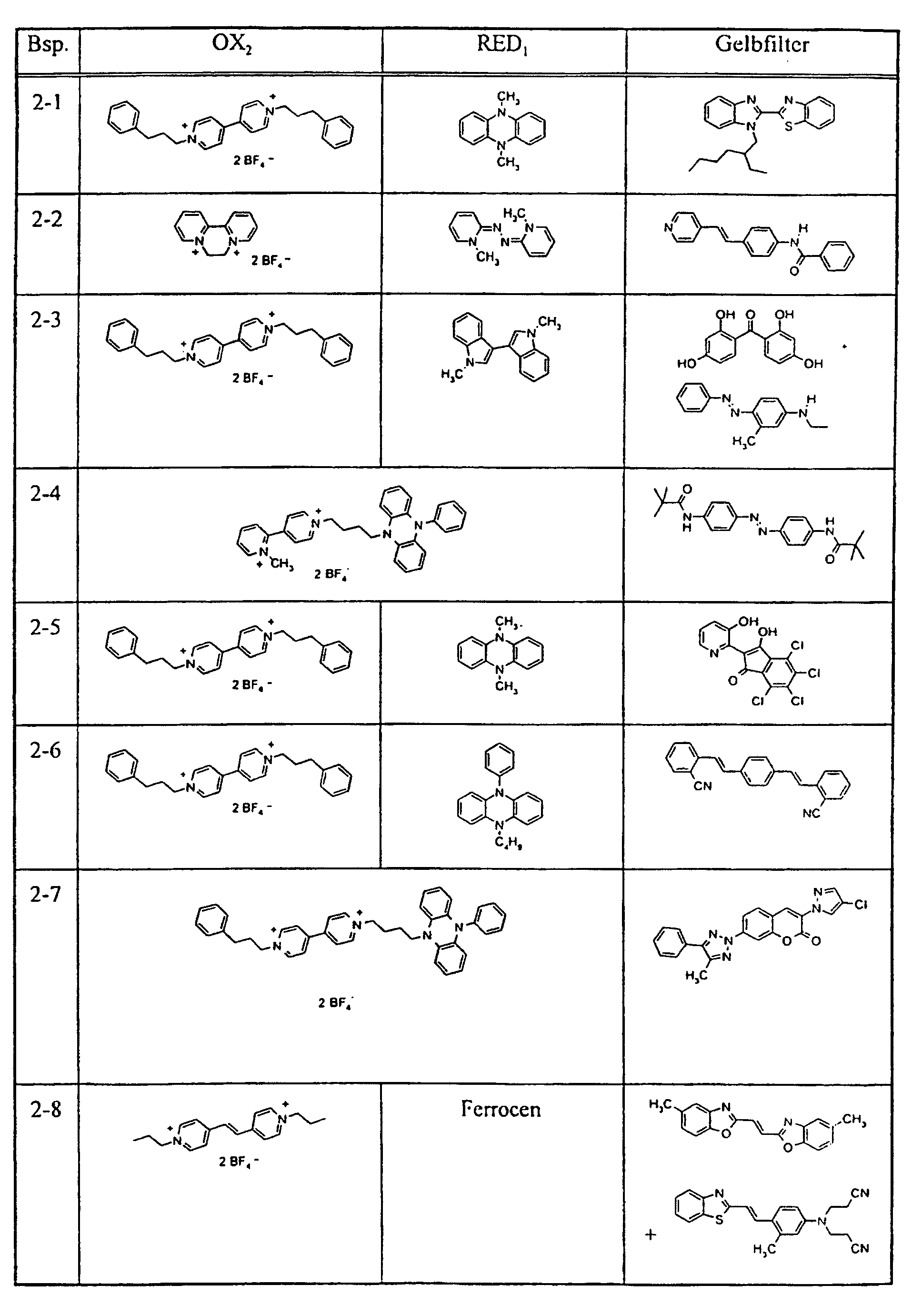

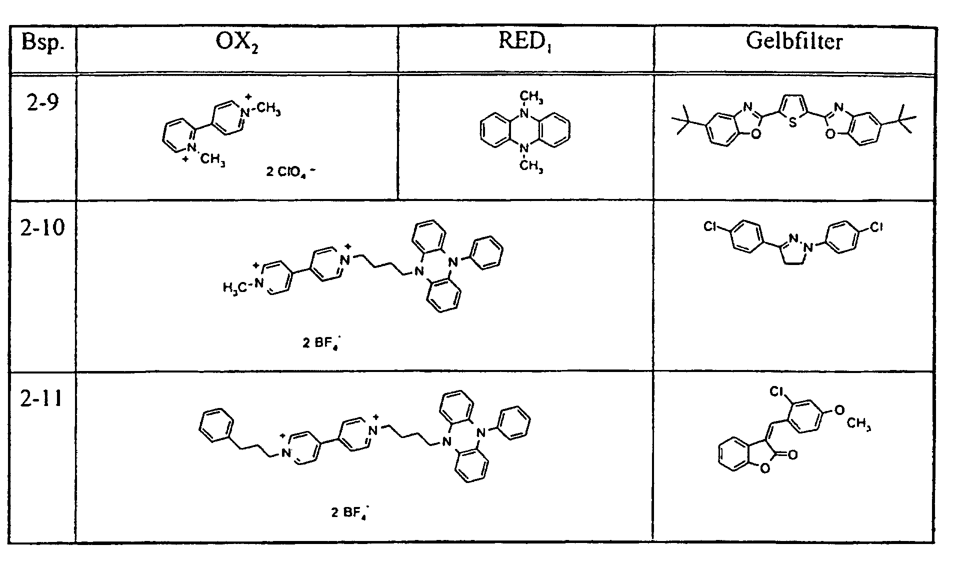

2-12: Es wurde eine Zelle gebaut, befüllt und verschlossen wie in Beispiel 2 beschrieben. Es wurden jedoch zwei Glasplatten benutzt, die auf der einen Seite mit ITO beschichtet waren und auf der anderen Seite wie folgt beschichtet waren: Eine 2 Gew.-%ige Lösung des polymeren Gelbfilters der Formel 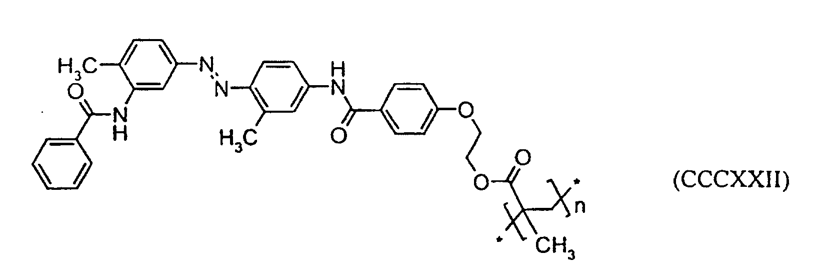 in Tetrahydrofuran wurde auf die nicht mit ITO beschichtete Seite der Platten aufgetragen und über Nacht bei 100°C getrocknet, so daß sich eine Trockenfilmdicke von 1 µm ergab.

### Beispiel 3

Es wurden wie in Beispiel 1 zwei Platten verwendet, auf einer Seite mit ITO beschichtet waren. Auf der anderen Seite wurde ein PU-Lack aus Desmodur und Desmophen (Bayer AG), der 3 Gewichtsprozent ZnO-Nanoteilchen und 1 Gewichtsprozent des Gelbfilters der Formel (CCCXXI) (s. Beispiel 2) enthielt, durch Rakeln in einer Naßfilmdicke von 240µm aufgebracht. Die Filme wurden über Nacht bei 130°C getrocknet.

Aus diesen Platten wird analog Beispiel 1 eine Zelle gebaut.

Die Zelle wurde mit einer Lösung gefüllt, die 0,02 molar an der elektrochromen Verbindung der Formel (CCC) (s. Beispiel 1) in wasserfreiem, sauerstofffreiem Propylencarbonat war. Die Zelle wurde wie in Beispiel 1 beschrieben verschlossen.

### Beispiel 3a (Vergleich)

Es wurde eine Zelle gebaut wie in Beispiel 3, wobei jedoch Glasplatten verwendet wurden, die nur mit ITO beschichtet waren.

### Lichtstabilitätstest

Die Zellen der Beispiele 3 (erfindungsagemäß) und 3a wurden, wie oben beschrieben, unter den gleichen Testbedingungen auf Lichtstabilität geprüft. Die Zelle gemäß Beispiel 3 war leichtechter als die Zelle gemäß Beispiel 3a.

### Beispiel 4

Es wurden wie in Beispiel 1 zwei Platten verwendet, die auf einer Seite mit ITO beschichtet waren. Auf der anderen Seite wurden sie mit einer CeO₂-Nanoteilchen beinhaltenden Schicht versehen. Die Schicht wurde folgendermaßen aufgebracht. Es wurde zuerst eine 6 Gew.-%ige Polyvinylalkohol-Lösung und eine 7 Gew.-%ige CeO₂-Dispersion jeweils in Wasser angesetzt. Die CeO₂-Nanoteilchen wurden von der Firma Rhodia, Frankfurt bezogen und weisen ein Größenverteilung von 8 ± 2nm auf. Diese Lösungen wurden 1:1 gemischt. Diese Mischung ist eine 4 Gew.-%ige Polyvinylalkohol-Lösung mit einem CeO₂-Anteil, der 20-Gew.-%, bezogen auf den Gesamtfeststoffanteil entspricht.

Die aufgebrachte Naßschicht hatte eine Schichtdicke von etwa 2 mm. Die nach einer Trocknung über Nacht bei Raumtemperatur resultierende Trockenschicht hatte eine Dicke von etwa 80 µm.

Aus diesen Platten wird analog Beispiel 1 eine Zelle gebaut.

Die Zelle wurde mit einer Lösung gefüllt, die 0,02 molar an der elektrochromen Verbindung der Formel 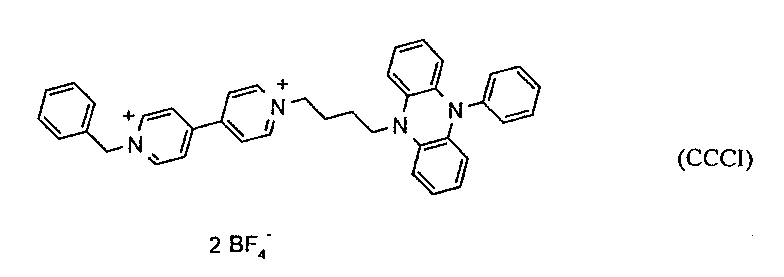 in wasserfreiem, sauerstofffreiem Propylencarbonat war. Die Zelle wurde wie in Beispiel 1 beschrieben verschlossen.

### Beispiel 4a (Vergleich)

Es wurde eine Zelle gebaut wie in Beispiel 4, wobei jedoch Glasplatten verwendet wurden, die nur mit ITO beschichtet waren.

### Lichtstabilitätstest

Die Zellen der Beispiele 4 (erfindungsgemäß) und 4a wurden, wie oben beschrieben, unter den gleichen Testbedingungen auf Lichtstabilität geprüft.

Der Vergleich mit den Zellen ohne Gelbfilter zeigt eine starke Erhöhung der Stabilität.

| In% | 0h | 0,5h | 1,5h | 3,5h | 7,5h | 15,5h | 39,5 | 87,5 | 183,5 |
|---|---|---|---|---|---|---|---|---|---|
| Zelle mit Gelbfilter | 100 | 100 | 100 | 100 | 97 | 96 | 88 | 81 | 49 |
| Zelle ohne Gelbfilter | 100 | 68 | 49 | 40 | 32 | - | - | - | - |

Die Schädigung der Zelle (20% weniger elektrochromer Hub) wurde nach 88 Stunden festgestellt. Gegenüber dem ungeschützten Zustand bedeutet dies eine Verbesserung um einen Faktor 250.

### Beispiel 5

Es wurde eine elektrochrome Zelle gebaut wie in Beispiel 1 beschrieben.

Die Zelle wurde mit einer Lösung gefüllt, die 0,02 molar an der elektrochromen Verbindung der Formel (CCC) (s. Beispiel 1) und 5 Gew.-% des Gelbfilters der Formel (CCCXXI) (s. Beispiel 2) in wasserfreiem, sauerstofffreiem Propylencarbonat war.

Die Zelle wurde wie in Beispiel 1 beschrieben verschlossen.

### Beispiel 5a (Vergleich)

Es wurde eine Zelle gebaut wie in Beispiel 5 beschrieben, jedoch ohne den Gelbfilter der Formel (CCCXXI).

### Lichtstabilitätstest

Die Zellen der Beispiele 5 (erfindungsagemäß) und 5a wurden, wie oben beschrieben, unter den gleichen Testbedingungen auf Lichtstabilität geprüft. Die Zelle gemäß Beispiel 5 war leichtechter als die Zelle gemäß Beispiel 5a.

## Patentansprüche

1. Lichtgeschützte elektrochrome Vorrichtung, enthaltend ein Paar Glas- oder Kunststoffplatten oder Kunststofffolien, von denen mindestens eine Platte oder Folie, vorzugsweise beide Platten oder Folien auf jeweils einer Seite mit einer elektrisch leitfähigen Beschichtung versehen sind, von denen wenigstens eine Platte oder Folie und ihre leitfähige Beschichtung transparent sind, von denen die andere verspiegelt sein kann und von denen wenigstens bei einer der beiden Platten oder Folien die elektrisch leitfähige Schicht in getrennte, einzeln kontaktierte Flächensegmente aufgeteilt sein kann, wobei die Platten oder Folien über einen Dichtungsring auf den Seiten ihrer leitfähigen Beschichtung zusammengefügt sind, und das Volumen, gebildet aus den beiden Platten oder Folien und dem Dichtungsring, mit einem elektrochromen Medium gefüllt ist, **dadurch gekennzeichnet, dass** die elektrochrome Vorrichtung einen Gelbfilter in dem elektrochromen Medium enthält, bei dem die Wellenlänge, bei der die Extinktion in der langwelligen Flanke die Hälfte des längstwelligen maximalen Extinktionswertes erreicht, zwischen 390 und 430 nm, liegt.

2. Lichtgeschützte elektrochrome Vorrichtung nach Anspruch 1, **dadurch gekennzeichnet, dass** als Gelbfilter spezielle Gläser oder oxidische oder keramische Beschichtungen verwendet werden.

3. Lichtgeschützte elektrochrome Vorrichtung nach Anspruch 1, **dadurch gekennzeichnet, dass** als Gelbfilter Nanoteilchen verwendet werden.

4. Lichtgeschützte elektrochrome Vorrichtung nach Anspruch 1, **dadurch gekennzeichnet, dass** neben dem Gelbfilter ein UV-absorbierendes Material verwendet wird, das die UV-Banden des Gelbfilters schützt.

5. Lichtgeschützte elektrochrome Vorrichtung nach Anspruch 9, **dadurch gekennzeichnet, dass** die UV-absorbierenden Materialien anorganische Nanoteilchen sind.

6. Lichtgeschützte elektrochrome Vorrichtung nach Anspruch 1, **dadurch gekennzeichnet, dass** im elektrochromen Medium mindestens eine Verbindung OX₂ der Formeln 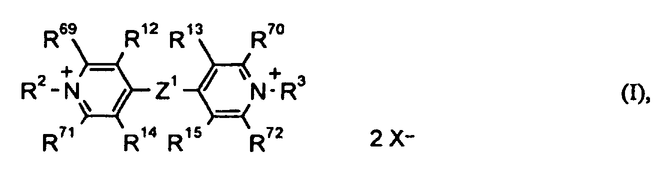 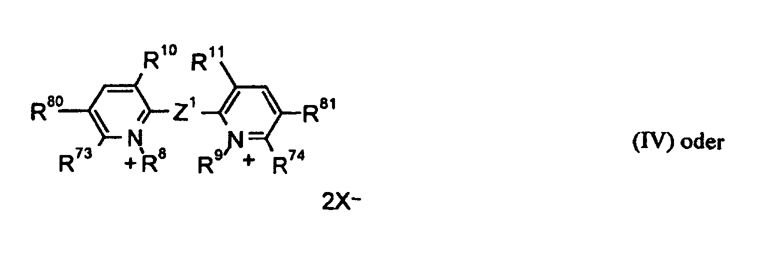 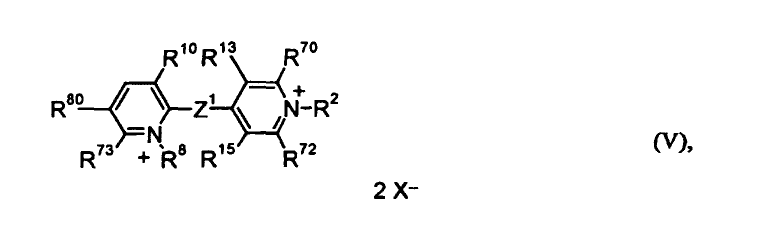 worin
R², R³, R⁸ und R⁹ unabhängig voneinander Methyl, Ethyl, Propyl, Butyl, Benzyl, Phenethyl, Phenylpropyl, Phenyl, 2-Methylphenyl oder 2,6-Dimethylphenyl bedeuten oder
R⁸ und R⁹ gemeinsam eine -(CH₂)₂- oder -(CH₂)₃-Brücke bilden,
R¹⁰ bis R¹⁵ Wasserstoff bedeuten,
R⁶⁹ bis R⁷³, R⁸⁰ und R⁸¹ unabhängig voneinander Wasserstoff oder Methyl bedeuten oder
R¹²; R⁶⁹, R¹³; R⁷⁰, R⁷³; R⁸⁰ und/oder R⁷⁴; R⁸¹ eine -CH=CH-CH=CH-Brücke bilden,
Z¹ eine direkte Bindung oder -CH=CH- bedeutet,
X⁻ ein unter den Bedingungen redox-inertes Anion bedeutet,
enthalten ist und mindestens eine Verbindung RED₁ der Formeln 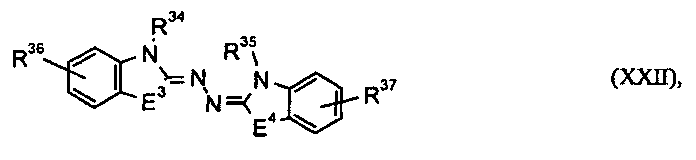 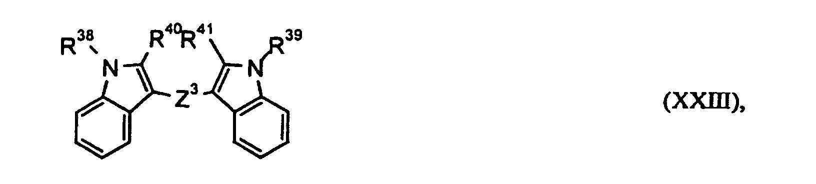 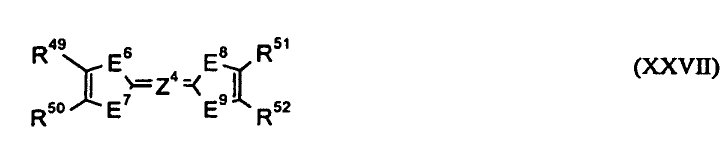 oder 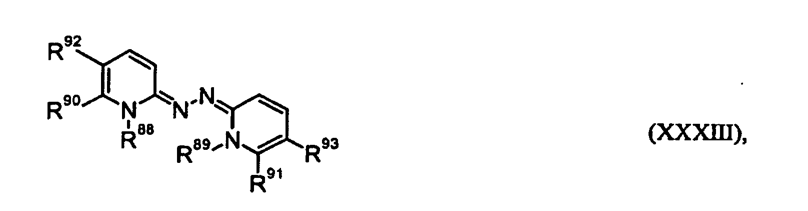 worin
R³⁴, R³⁵, R³⁸, R³⁹, R⁸⁸ und R⁸⁹ unabhängig voneinander Methyl, Ethyl, Propyl, Butyl, Benzyl, Phenethyl, Phenylpropyl oder Phenyl bedeuten,
R³⁶ und R³⁷ Wasserstoff bedeuten,
Z³ eine direkte Bindung oder eine -CH=CH-Brücke bedeutet,
Z⁴ eine direkte Doppelbindung bedeutet,
R⁴⁰ und R⁴¹ gleich sind und Wasserstoff oder Methyl bedeuten,
E³ und E⁴ gleich sind und S, N-R⁵⁹ oder C(CH₃)₂ bedeuten,
E⁶ bis E⁹ gleich sind und S bedeuten,
R⁴⁹ bis R⁵² unabhängig voneinander Wasserstoff, Methyl, Cyano oder Methoxycarbonyl bedeuten oder
R⁴⁹; R⁵⁰ und/oder R⁵¹; R⁵² eine -(CH₂)₃- oder -CH=CH-CH=CH-Brücke bilden,
R⁹⁰ bis R⁹³ Wasserstoff bedeuten oder
R⁹⁰; R⁹² und/oder R⁹¹; R⁹³ eine -CH=CH-CH=CH-Brücke bilden und
R⁵⁹ Methyl, Ethyl, Propyl oder Butyl bedeutet,
enthalten ist, wobei OX₂ und RED₁ über eine Brücke B gemäß der Formel
OX₂B-RED₁ (La)
verknüpft sein können,
worin
B -(CH₂)ₙ- bedeutet und
n eine ganze Zahl von 3 bis 6 bedeutet.

7. Verwendung der lichtgeschützten elektrochromen Vorrichtung nach Anspruch 1 als Fenster oder Trennscheibe oder Sichtschutzscheibe oder Verglasung oder Dachverglasung.

8. Elektrochrome Substanzen der Formel 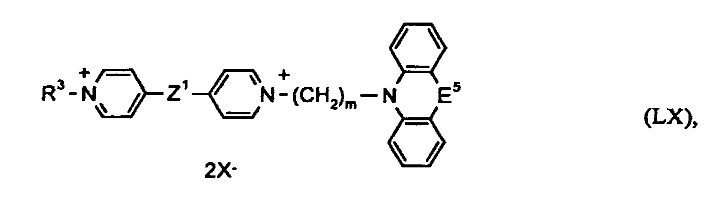 worin
R³ -(CH₂)₃-C₆H₅ oder -(CH₂)₄-C₆H₅ bedeutet,
Z¹ eine direkte Bindung bedeutet,
E⁵ N-C₆H₅, N-(CH₂)₃-C₆H₅ oder N-(CH₂)₄-C₆H₅ bedeutet,
m eine ganze Zahl von 3 bis 5 bedeutet und
X⁻ ein unter den Bedingungen redox-inertes Anion bedeutet.

9. Elektrochrome Substanz der Formel

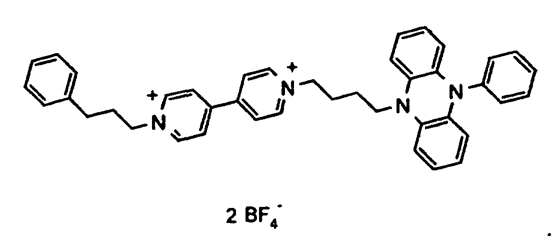

10. Verwendung eines Gelbfilters, bei dem die Wellenlänge bei der die Extinktion in der langwelligen Flanke die Hälfte des längstwelligen maximalen Extinktionswertes erreicht, zwischen 390 und 430 nm liegt, zur Erhöhung der Lichtbeständigkeit von elektrochromen Verbindungen im geschalteten Zustand.

## Claims

1. Light-stabilised electrochromic device comprising a pair of glass or plastics material sheets or plastics material films, of which at least one sheet or film, preferably the two sheets or films, is provided on one side each with an electrically conductive coating, of which at least one sheet or film and its conductive coating are transparent, of which the other one can be mirrored, and of which at least in one of the two sheets or films the electrically conductive layer can be divided into separate, individually contacted area segments, the sheets or films being joined by a ring seal on the sides of their conductive coating, and the volume formed by the two sheets or films and the ring seal being filled with an electrochromic medium, **characterised in that** the electrochromic device comprises a yellow filter in the electrochromic medium for which the wavelength at which the absorbance in the long-wavelength flank reaches half of the longest wavelength maximum absorbance is between 390 and 430 nm.

2. Light-stabilised electrochromic device according to claim 1, **characterised in that** special glasses or oxidic or ceramic coatings are used as the yellow filter.

3. Light-stabilised electrochromic device according to claim 1, **characterised in that** nanosize particles are used as the yellow filter.

4. Light-stabilised electrochromic device according to claim 1, **characterised in that**, in addition to the yellow filter, a UV-absorbing material that protects the UV bands of the yellow filter is used.

5. Light-stabilised electrochromic device according to claim 1, **characterised in that** the UV-absorbing materials are inorganic nanosize particles.

6. Light-stabilised electrochromic device according to claim 1, **characterised in that** the electrochromic medium contains at least one compound OX₂ of the formulae 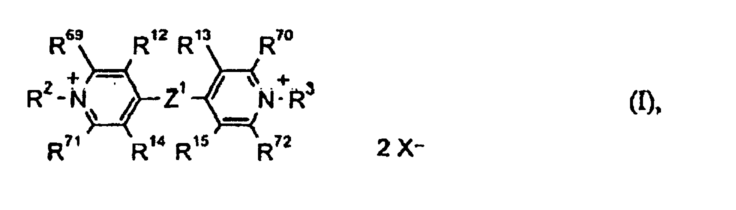 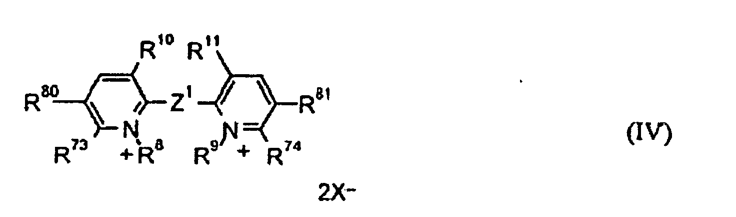 or 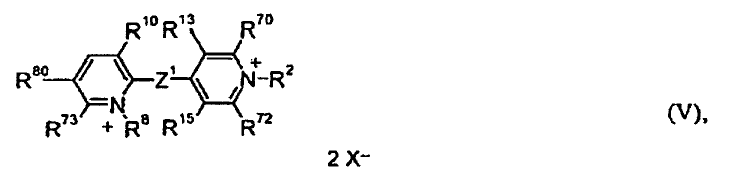 wherein
R², R³, R⁸ and R⁹, independently of one another, are methyl, ethyl, propyl, butyl, benzyl, phenethyl, phenylpropyl, phenyl, 2-methylphenyl, or 2,6-dimethylphenyl or
R⁸ and R⁹ together form a -(CH₂)₂- or -(CH₂)₃- bridge,
R¹⁰ to R¹⁵ are hydrogen,
R⁶⁹ to R⁷³, R⁸⁰ and R⁸¹, independently of one another, are hydrogen or methyl or
R¹² and R⁶⁹ together, R¹³ and R⁷⁰ together, R⁷³ and R⁸⁰ together, and/or R⁷⁴ and R⁸¹ together form a -CH=CH-CH=CH- bridge,
Z¹ is a direct bond or -CH=CH-,
X⁻ is an anion that is redox-inert under the conditions,
and at least one compound RED₁ of the formulae 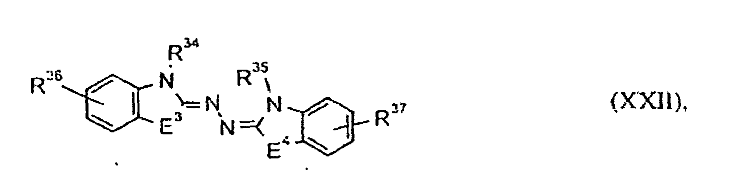 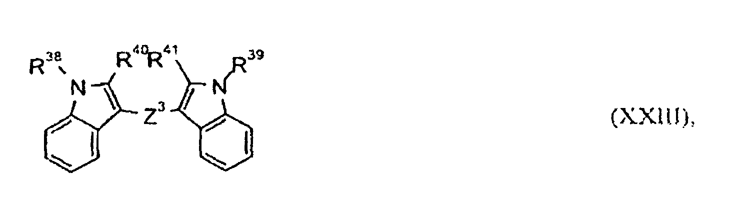 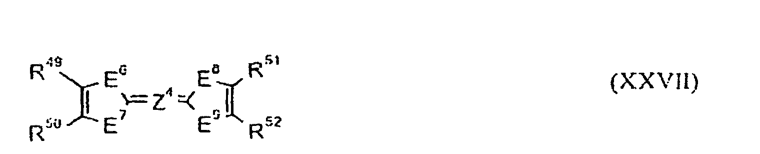 or 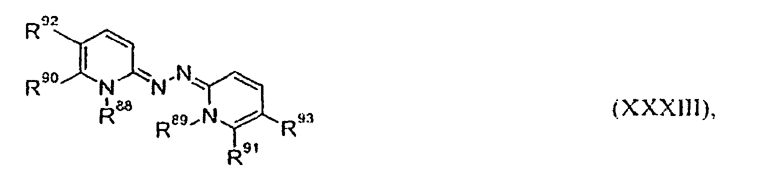 wherein
R³⁴, R³⁵, R³⁸, R³⁹, R⁸⁸ and R⁸⁹, independently of one another, are methyl, ethyl, propyl, butyl, benzyl, phenethyl, phenylpropyl, or phenyl,
R³⁶ and R³⁷ are hydrogen,
Z³ is a direct bond or a -CH=CH- bridge,
Z⁴ is a direct double bond,
R⁴⁰ and R⁴¹ are the same and are hydrogen or methyl,
E³ and E⁴ are the same and are S, N-R⁵⁹, or C(CH₃)₂,
E⁶ to E⁹ are the same and are S,
R⁴⁹ to R⁵², independently of one another, are hydrogen, methyl, cyano, or methoxycarbonyl or
R⁴⁹ and R⁵⁰ together and/or R⁵¹ and R⁵² together form a -CH=CH-CH=CH- bridge,
R⁹⁰ to R⁹³ are hydrogen or
R⁹⁰ and R⁹² together and/or R⁹¹ and R⁹³ together form a -CH=CH-CH=CH- bridge, and
R⁵⁹ is methyl, ethyl, propyl or butyl,
wherein OX₂ and RED₁ can be linked via a bridge B according to the formula
OX₂-B-RED₁ (La),
wherein
B is -(CH₂)n- and
n is an integer from 3 to 6.

7. Use of the light-stabilised electrochromic device according to claim 1 as a window, partition, visor, glazing or skylight.

8. Electrochromic substances of the formula 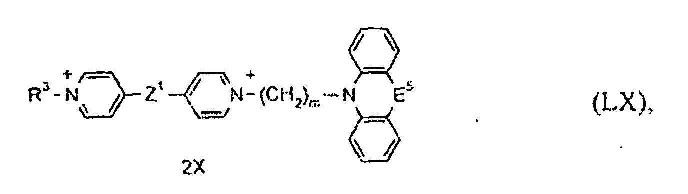 wherein
R³ is -(CH₂)₃-C₆H₅ or -(CH₂)₄-C₆H₅,
Z¹ is a direct bond,
E⁵ is N-C₆H₅, N-(CH₂)₃-C₆H₅ or N-(CH₂)₄-C₆H₅,
m is an integer from 3 to 5 and
X⁻ is an anion which is redox-inert under the conditions.

9. Electrochromic substance of the formula

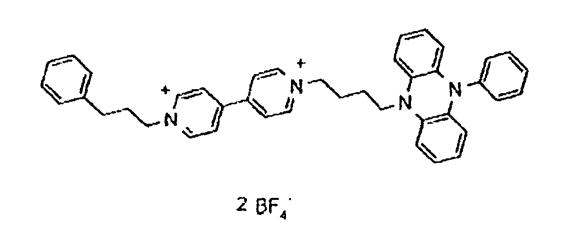

10. Use of a yellow filter, for which the wavelength, at which the absorbance in the long-wavelength flank reaches half of the longest wavelength maximum absorbance, is between 390 and 430 nm, to increase the light resistance of electrochromic compounds in the connected state.

## Revendications

1. Dispositif électrochromique protégé contre la lumière, contenant une paire de plaques en verre ou en matière plastique ou de feuilles plastiques, dont au moins une plaque ou feuille, de préférence les deux plaques ou feuilles, sont munies, chaque fois sur une face, d'un revêtement conduisant l'électricité, dont au moins une plaque ou feuille et son revêtement conducteur sont transparents, dont l'autre peut être métallisée et dont au moins pour une des deux plaques ou feuilles, la couche conduisant l'électricité peut être séparée en segments de surface séparés, mis individuellement en contact, où les plaques ou feuilles sont montées par un joint étanche sur la face de leur revêtement conducteur, et le volume, formé par les deux plaques ou feuilles et le joint étanche, est rempli d'un milieu électrochrome, **caractérisé en ce que** le dispositif électrochromique contient un filtre jaune dans le milieu électrochrome, pour lequel la longueur d'onde, à laquelle l'extinction dans le flanc de longue longueur d'onde atteint la moitié de la valeur d'extinction maximale aux ondes les plus longues, se situe entre 390 et 430 nm.

2. Dispositif électrochromique protégé contre la lumière selon la revendication 1, **caractérisé en ce que** l'on utilise comme filtre jaune, des verres spéciaux ou des revêtements oxydes ou céramiques.

3. Dispositif électrochromique protégé contre la lumière selon la revendication 1, **caractérisé en ce que** l'on utilise comme filtre jaune, des nanoparticules.

4. Dispositif électrochromique protégé contre la lumière selon la revendication 1, **caractérisé en ce que** l'on utilise en plus du filtre jaune, un matériau absorbant les U.V., qui protège les bandes U.V. du filtre jaune.

5. Dispositif électrochromique protégé contre la lumière selon la revendication 9, **caractérisé en ce que** les matériaux absorbant les U.V. sont des nanoparticules inorganiques.

6. Dispositif électrochromique protégé contre la lumière selon la revendication 1, **caractérisé en ce que** dans le milieu électrochrome, est présent au moins un composé OX₂ des formules : 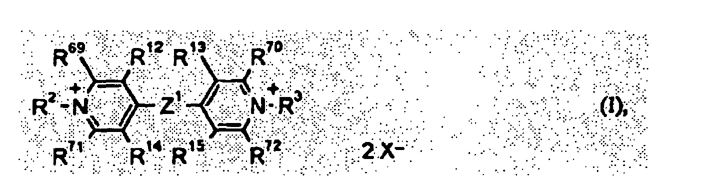 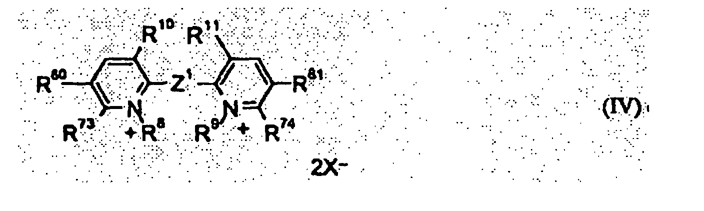 ou 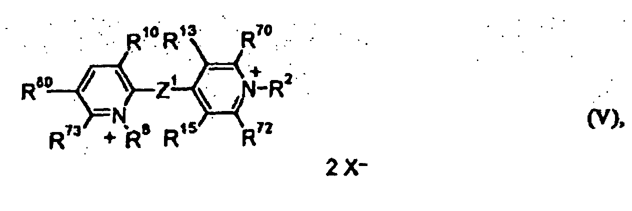 où
R²,R³, R⁸ et R⁹ signifient indépendamment l'un de l'autre, méthyle, éthyle, propyle, butyle, benzyle, phénéthyle, phénylpropyle, phényle, 2-méthylphényle ou 2,6-diméthylphényle, ou
R⁸ et R⁹ forment ensemble, un pont (CH₂)₂- ou - (CH₂)₃-,
R¹⁰ à R¹⁵ signifient hydrogène,
R⁶⁹ à R⁷³ , R⁸⁰ et R⁸¹ signifient indépendamment l'un de l'autre, hydrogène ou méthyle, ou
R¹², R⁶⁹; R¹³, R⁷⁰; R⁷³, R⁸⁰ et/ou R⁷⁴, R⁸¹ forment un pont -CH=CH-CH=CH-,
Z¹ signifie une liaison directe ou -CH=CH-,
X⁻ signifie un anion inerte dans des conditions rédox,
et au moins un composé RED₁ des formules : 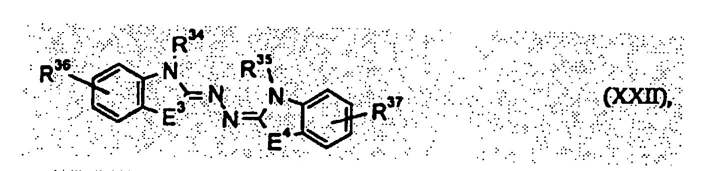 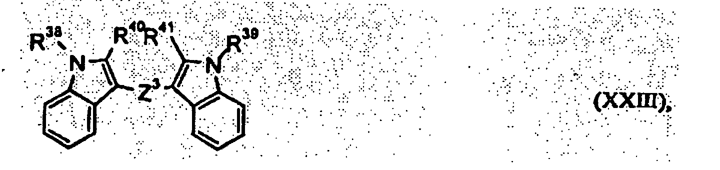 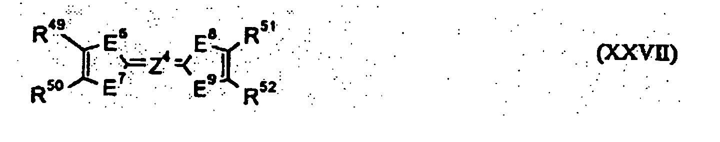 où 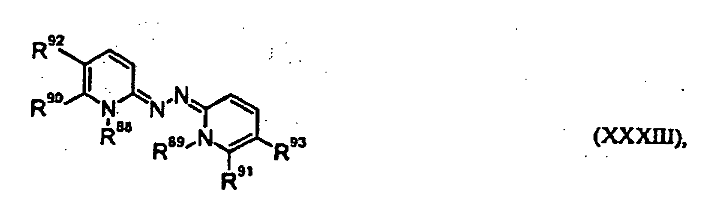 où
R³⁴, R³⁵, R³⁸, R³⁹, R⁸⁸ et R⁸⁹ signifient indépendamment l'un de l'autre, méthyle, éthyle, propyle, butyle, benzyle, phénéthyle, phénylpropyle ou phényle,
R³⁶ et R³⁷ signifient hydrogène,
Z³ signifie une liaison directe ou -CH=CH-,
Z⁴ signifie une double liaison directe,
R⁴⁰ et R⁴¹ sont identiques et signifient hydrogène ou méthyle,
E³ et E⁴ sont identiques et signifient S, N-R⁵⁹ ou C (CH₃) ₂,
E⁶ à E⁹ sont identiques et signifient S,
R⁴⁹ à R⁵² signifient indépendamment l'un de l'autre, hydrogène, méthyle, cyano ou méthoxycarbonyle, ou
R⁴⁹, R⁵⁰ et/ou R⁵¹, R⁵² forment un pont (CH₂) ₃ ou -CH=CH-CH=CH-,
R⁹⁰ à R⁹³ signifient hydrogène, ou
R⁹⁰, R⁹² et/ou R⁹¹, R⁹³ forment un pont -CH=CH-CH=CH-,
R⁵⁹ signifie méthyle, éthyle, propyle ou butyle,
où OX₂ et RED₁ peuvent être reliés par un pont B selon la formule :
OX₂ - B - RED₁ (La)
où
B signifie -(CH₂)ₙ-, et
n signifie un nombre entier allant de 3 à 6.

7. Utilisation du dispositif électrochromique protégé contre la lumière selon la revendication 1, comme fenêtre ou pare-brise arrière ou oculaire de protection ou vitrage ou vitrage de toit.

8. Substances électrochromes de la formule : 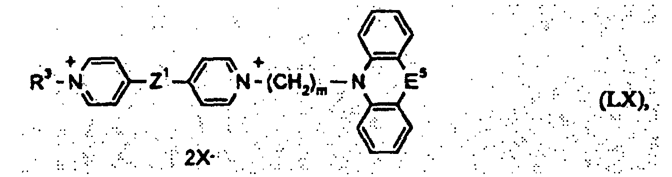 où
R³ signifie -(CH₂)₃-C₆H₅ ou -(CH₂)₄-C₆H₅,
Z¹ signifie une liaison directe,
E⁵ signifie N-C₆H₅, N-(CH₂)₃-C₆H₅ ou N-(CH₂)₄-C₆H₅,
m signifie un nombre entier allant de 3 à 5, et
X⁻ signifie un anion inerte dans des conditions rédox.

9. substance électrochrome de la formule :

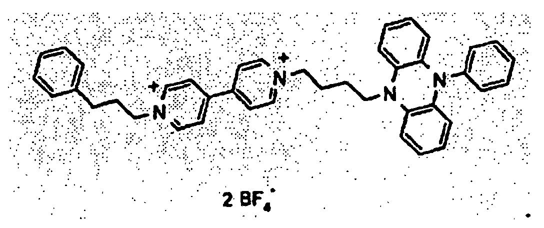

10. Utilisation d'un filtre jaune, dans lequel la longueur d'onde à laquelle l'extinction dans le flanc de longue longueur d'onde atteint la moitié de la valeur d'extinction maximale aux plus longues longueurs d'onde, se situe entre 390 et 430 nm, pour augmenter la résistance à la lumière de dispositifs électrochromiques en circuit.
